# EUROPEAN PATENT SPECIFICATION

(11) **EP 1 135 512 B1**
(45) Date of publication and mention of the grant of the patent: **13.10.2004**
(21) Application number: 99961833.3
(22) Date of filing: 30.11.1999
(51) Int. Cl.: C12N 15/82, C12N 15/29, C12N 15/67, C12N 15/63, C12N 5/10

(54) **ARTIFICIAL MATRIX ATTACHMENT REGION FOR INCREASING EXPRESSION OF GENES INTRODUCED IN PLANT CELLS**
KÜNSTLICHE MATRIX-ANHEFTUNGSREGION ZUR ERHÖHUNG DER EXPRESSION VON IN PFLANZEN EINGEFÜHRTEN GENEN
REGION DE FIXATION DE MATRICE ARTIFICIELLE DESTINEE A AUGMENTER L'EXPRESSION DE GENES INTRODUITS DANS DES CELLULES VEGETALES

(30) Priority: 01.12.1998 US 110437 P
(43) Date of publication of application: 26.09.2001
(73) Proprietor: Dow AgroSciences LLC, Indianapolis, IN 46268 (US)
(72) Inventor: VAN DER GEEST, Apolonia, H.M., NL-6708 KX Wageningen (NL); AINLEY, W., Michael, Carmel, IN 46032 (US); COWEN, Neil, M., Zionsville, IN 46077 (US); WELTER, Mary, E., Indianapolis, IN 46220 (US); WOOSLEY, Aaron, T., Fishers, IN 46038 (US)
(74) Representative: Smart, Peter John
(86) International application number: PCT/US1999/028123
(87) International publication number: WO 2000/032800

(56) References cited:
- WO-A-97/27207
- US-A- 5 773 689
- LIU J -W ET AL: "THE INFLUENCE OF TWO PLANT NUCLEAR MATRIX ATTACHMENT REGIONS (MARS) ON GENE EXPRESSION IN TRANSGENIC PLANTS" MOLECULAR AND GENERAL GENETICS,DE,SPRINGER VERLAG, BERLIN, vol. 39, no. 1, 1 January 1998 (1998-01-01), pages 115-123, XP002910494 ISSN: 0026-8925
- BREYNE P ET AL: "Characterisation of a plant scaffold attachemt region in a DNA fragment that normalises transgene expression in tobacco" PLANT CELL,US,AMERICAN SOCIETY OF PLANT PHYSIOLOGISTS, ROCKVILLE, MD, no. 4, 1 April 1992 (1992-04-01), pages 463-471, XP002072399 ISSN: 1040-4651
- RIPOLL P -J ET AL: "A new yeast artificial chromosome vector designed for gene transfer into mammalian cells" GENE: AN INTERNATIONAL JOURNAL ON GENES AND GENOMES,GB,ELSEVIER SCIENCE PUBLISHERS, BARKING, vol. 210, no. 1, 27 March 1998 (1998-03-27), pages 163-172, XP004117463 ISSN: 0378-1119
- VON KRIES J P ET AL: "A MATRIX-SCAFFOLD ATTACHMENT REGION BINDING PROTEIN IDENTIFICATION PURIFICATION AND MODE OF BINDING" CELL 1991, vol. 64, no. 1, 1991, pages 123-136, XP000891516 ISSN: 0092-8674 cited in the application
- STEIN G S ET AL: "REGULATION OF TRANSCRIPTION-FACTOR ACTIVITY DURING GROWTH AND DIFFERENTIATION INVOLVEMENT OF THE NUCLEAR MATRIX IN CONCENTRATION AND LOCALIZATION OF PROMOTER BINDING PROTEINS" JOURNAL OF CELLULAR BIOCHEMISTRY 1991, vol. 47, no. 4, 1991, pages 300-305, XP000864497 ISSN: 0730-2312 cited in the application
- SANDER M ET AL: "Drosophila topoisomerase II double-strand DNA cleavage: analysis of DNA sequence homology at the cleavage site." NUCLEIC ACIDS RESEARCH, (1985 FEB 25) 13 (4) 1057-72. , XP000891514 cited in the application

### SEQUENCE LISTING

<110> van der Geest, Apolonia H. M.
   Ainley, W. Michael
   Cowen, Neil W.
   Welter, Mary E.
   Woosley, Aaron T
<120> ARTIFICIAL MATRIX ATTACHMENT REGION FOR INCREASING EXPRESSION OF GENES INTRODUCED IN PLANT CELLS
<130> 50545
<140>
   <141>
<150> US 60/110,437
   <151> 1998-12-01
<160> 33
<170> PatentIn Ver. 2.0
<210> 1
   <211> 327
   <212> DNA
   <213> Artificial Sequence
<220>
   <223> Description of Artificial Sequence: artificial MAR
<400> 1
   ttccagatct cgatatcttt aatttctaat atatttagaa ggggtttaga tttatatatg 60
   acgtccatga aaaaaaattt taaaacgata ggccagctcc aaagaatata tttccctgga 120
   gctggtaaat attaattagt cctctccctt ctaatatatt tttcgatatt tttgattcct 180
   ttttaagatt atatagctcc atgccaagct gacttcctaa tatattttat gaagtcagca 240
   aaatttttaa aaagcacact tgacttggta tatatttata aatgtttaaa cttaatttca 300
   cttgtaaaac tcttgcagga tccgtgc                                     327
<210> 2
   <211> 15
   .<212> DNA
   <213> Artificial Sequence
<220>
   <223> Description of Artificial Sequence: ARBP site
<400> 2
   atttcasttg taaaa    15
<210> 3
   <211> 13
   <212> DNA
   <213> Artificial Sequence
<220>
   <223> Description of Artificial Sequence: ARBP site
<400> 3
   tgcaggtgtc ctt    13
<210> 4
   <211> 13
   <212> DNA
   <213> Artificial Sequence
<220>
   <223> Description of Artificial Sequence: ARBP site
<400> 4
   tgggggtgta aaa    13
<210> 5
   <211> 11
   <212> DNA
   <213> Artificial Sequence
<220>
   <223> Description of Artificial Sequence: ATF site
<400> 5
   tgacgtccat g 11
<210> 6
   <211> 5
   <212> DNA
   <213> Artificial Sequence
<220>
   <223> Description of Artificial Sequence: BEAF-32 site
<400> 6
   cgata 5
<210> 7
   <211> 13
   <212> DNA
   <213> Artificial Sequence
<220>
   <223> Description of Artificial Sequence: topoisomerase II site
<400> 7
   cnngyngktn yny 13
<210> 8
   <211> 16
   <212> DNA
   <213> Artificial Sequence
<220>
   <223> Description of Artificial Sequence: topoisomerase II site
<400> 8
   asmatgcgyw yatcrt 16
<210> 9
   <211> 15
   <212> DNA
   <213> Artificial Sequence
<220>
   <223> Description of Artificial Sequence: topoisomerase II site
<400> 9
   gtnwakattn atnnr 15
<210> 10
   <211> 9
   <212> DNA
   <213> Artificial Sequence
<220>
   <223> Description of Artificial Sequence: unwinding sequence
<400> 10
   aatatattt 9
<210> 11
   <211> 11
   <212> DNA
   <213> Artificial Sequence
<220>
   <223> Description of Artificial Sequence: SATB1 site
<400> 11
   ttctaatata t 11
<210> 12
   <211> 9
   <212> DNA
   <213> Artificial Sequence
<220>
   <223> Description of Artificial Sequence: SATB1 site
<400> 12
   ataatcttc 9
<210> 13
   <211> 10
   <212> DNA
   <213> Artificial Sequence
<220>
   <223> Description of Artificial Sequence: SATB1 site
<400> 13
   ttattattta 10
<210> 14
   <211> 8
   <212> DNA
   <213> Artificial Sequence
<220>
   <223> Description of Artificial Sequence: SATB1 site
<400> 14
   tataaaaa 8
<210> 15
   <211> 11
   <212> DNA
   <213> Artificial Sequence
<220>
   <223> Description of Artificial Sequence: SATB1 site
<400> 15
   aagattatat a 11
<210> 16
   <211> 17
   <212> DNA
   <213> Artificial Sequence
<220>
   <223> Description of Artificial Sequence: SATB1 site
<400> 16
   ttttaatgag ataataa 17
<210> 17
   <211> 10
   <212> DNA
   <213> Artificial Sequence
<220>
   <223> Description of Artificial Sequence: SATB1 site
<400> 17
   tataatcttc 10
<210> 18
   <211> 13
   <212> DNA
   <213> Artificial Sequence
<220>
   <223> Description of Artificial Sequence: bending DNA site
<400> 18
   aaannnnnnn aaa 13
<210> 19
   <211> 7
   <212> DNA
   <213> Artificial Sequence
<220>
   <223> Description of Artificial Sequence: exemplary oligo A/T tract
<400> 19
   aaaaaaa 7
<210> 20
<211> 54
   <212> DNA
   <213> Artificial Sequence
<220>
   <223> Description of Artificial Sequence: MAR-A oligonucleotide
<400> 20
   ttccagatct cgatatcttt aatttctaat atatttagaa ggggtttaga ttta 54
<210> 21
   <211> 63
   <212> DNA
   <213> Artificial Sequence
<220>
   <223> Description of Artificial Sequence: MAR-B oligonucleotide
<400> 21
   gaaggggttt agatttatat atgacgtcca tgaaaaaaaa ttttaaaacg ataggccagc 60
   tcc 63
<210> 22
   <211> 63
   <212> DNA
   <213> Artificial Sequence
<220>
   <223> Description of Artificial Sequence: MAR-C oligonucleotide
<400> 22
   cgataggcca gctccaaaga atatatttcc ctggagctgg taaatattaa ttagtcctct 60
   ccc 63
<210> 23
   <211> 75
   <212> DNA
   <213> Artificial Sequence
<220>
   <223> Description of Artificial Sequence: MAR-D oligonucleotide
<400> 23
   cttggcatgg agctatataa tcttaaaaag gaatcaaaaa tatcgaaaaa tatattagaa 60
   gggagaggac taatt                                                  75
<210> 24
   <211> 74
   <212> DNA
   <213> Artificial Sequence
<220>
   <223> Description of Artificial Sequence: MAR-E oligonucleotide
<400> 24
   ccaagtcaag tgtgcttttt aaaaattttg ctgacttcat aaaatatatt aggaagtcag 60
   cttggcatgg agct                                                   74
<210> 25
   <211> 74
   <212> DNA
   <213> Artificial Sequence
<220>
   <223> Description of Artificial Sequence: MAR-F oligonucleotide
<400> 25
   gcacggatcc tgcaagagtt ttacaagtga aattaagttt aaacatttat aaatatatac 60
   caagtcaagt gtgc                                                   74
<210> 26
   <211> 668
   <212> DNA
   <213> Artificial Sequence
<220>
   <223> Description of Artificial Sequence: 3' MAR in ArActAf
<400> 26
   gggggatctc gatatcttta atttctaata tatttagaag gggtttagat ttatatatga 60
   cgtccatgaa aaaaaatttt aaaacgatag gccagctcca aagaatatat ttccctggag 120
   ctggtaaata ttaattagtc ctctcccttc taatatattt ttcgatattt ttgattcctt 180
   tttaagatta tatagctcca tgccaagctg acttcctaat atattttatg aagtcagcaa 240
   aatttttaaa aagcacactt gacttggtat atatttataa atgtttaaac ttaatttcac 300
   ttgtaaaact cttgcaggat ctcgatatct ttaatttcta atatatttag aaggggttta 360
   gatttatata tgacgtccat gaaaaaaaat tttaaaacga taggccagct ccaaagaata 420
   tatttccctg gagctggtaa atattaatta gtcctctccc ttctaatata tttttcgata 480
   tttttgattc ctttttaaga ttatatagct ccatgccaag ctgacttcct aatatatttt 540
   atgaagtcag caaaattttt aaaaagcaca cttgacttgg tatatattta taaatgttta 600
   aacttaattt cacttgtaaa actcttgcag gatccactag ttctagagcg gccgccaccg 660
   cggtggag                                                          668
<210> 27
   <211> 9361
   <212> DNA
   <213> Artificial Sequence
<220>
   <223> Description of Artificial Sequence:pGOS2-hpt
<400> 27
   gshttcgcgc gtttcggtga tgacggtgaa aacctctgac acatgcagct cccggagacg 60
   gtcacagctt gtctgtaagc ggatgccggg agcagacaag cccgtcaggg cgcgtcagcg 120
   ggtgttggcg ggtgtcgggg ctggcttaac tatgcggcat cagagcagat tgtactgaga 180
   gtgcaccata tgcggtgtga aataccgcac agatgcgtaa ggagaaaata ccgcatcagg 240
   cgccattcgc cattcaggct gcgcaactgt tgggaagggc gatcggtgcg ggcctcttcg 300
   ctattacgcc agctggcgaa agggggatgt gctgcaaggc gattaagttg ggtaacgcca 360
   gggttttccc agtcacgacg ttgtaaaacg acggccagtg aattcccgat cgatctagta 420
   acatagatga caccgcgcgc gataatttat cctagtttgc gcgctatatt ttgttttcta 480
   tcgcgtatta aatgtataat tgcgggactc taatcataaa aacccatctc ataaataacg 540
   tcatgcatta catgttaatt attacatgct taacgtaatt caacagaaat tatatgataa 600
   tcatcgcaag accggcaaca ggattcaatc ttaagaaact ttattgccaa atgtttgaac 660
   gatcggggaa attcgagctc ttactattcc tttgccctcg gacgagtgct ggggcgtcgg 720
   tttccactat cggcgagtac ttctacacag ccatcggtcc agacggccgc gcttctgcgg 780
   gcgatttgtg tacgcccgac agtcccggct ccggatcgga cgattgcgtc gcatcgaccc 840
   tgcgcccaag ctgcatcatc gaaattgccg tcaaccaagc tctgatagag ttggtcaaga 900
   ccaatgcgga gcatatacgc ccggagccgc ggcgatcctg caagctccgg atgcctccgc 960
   tcgaagtagc gcgtctgctg ctccatacaa gccaaccacg gcctccagaa gaagatgttg 1020
   gcgacctcgt attgggaatc cccgaacatc gcctcgctcc agtcaatgac cgctgttatg 1080
   cggccattgt ccgtcaggac attgttggag ccgaaatccg cgtgcacgag gtgccggact 1140
   tcggggcagt cctcggccca aagcatcagc tcatcgagag cctgcgcgac ggacgcactg 1200
   acggtgtcgt ccatcacagt ttgccagtga tacacatggg gatcagcaat cgcgcatatg 1260
   aaatcacgcc atgtagtgta ttgaccgatt ccttgcggtc cgaatgggcc gaacccgctc 1320
   gtctggctaa gatcggccgc agcgatcgca tccatggcct ccgcgaccgg ctgcagaaca 1380
   gcgggcagtt cggtttcagg caggtcttgc aacgtgacac cctgtgcacg gcgggagatg 1440
   caataggtca ggctctcgct gaattcccca atgtcaagca cttccggaat cgggagcgcg 1500
   gccgatgcaa agtgccgata aacataacga tctttgtaga aaccatcggc gcagctattt 1560
   acccgcagga catatccacg ccctcctaca tcgaagctga aagcacgaga ttcttcgccc 1620
   tccgagagct gcatcaggtc ggagacgctg tcgaactttt cgatcagaaa cttctcgaca 1680
   gacgtcgcgg tgagttcagg ctttttcata tctcacgaga tctgtatccc gtgcctggaa 1740
   caaatggccc cagatccgtc cgcagctgca cgggtccagg aaagcaatcg catagtcaag 1800
   ctaaatcatc aagatgcaaa cttttcgccc ttgctaaaca cggtaaaatt cgaatggaca 1860
   tgtgtggagc agcaaaggag ctttccccaa aattactcaa cgaatcataa accaagatta 1920
   gtcagatcaa gagacagagg agaaacaagg cggacctttg cacttgatct ggggattgcc 1980
   ctgacttggt ggtgctggta tattagggat agggttgctc ctatccacag cttgtccacc 2040
   aaatatcagc tcctccgtgg actgccttgt cgagccttca gctggatcct ctagagtccc 2100
   ccgtgttctc tccaaatgaa atgaacttcc ttatatagag gaagggtctt gcgaaggata 2160
   gtgggattgt gcgtcatccc ttacgtcagt ggagatatca catcaatcca cttgctttga 2220
   agacgtggtt ggaacgtctt ctttttccac gatgctcctc gtgggtgggg gtccatcttt 2280
   gggaccactg tcggcagagg catcttcaac gatggccttt cctttatcgc aatgatggca 2340
   tttgtaggag ccaccttcct tttccactat cttcacaata aagtgacaga tagctgggca 2400
   atggaatccg aggaggtttc cggatattac cctttgttga aaagtctcca tcgatgatca 2460
   catcaatcca cttgctttga agacgtggtt ggaacgtctt ctttttccac gatgctcctc 2520
   gtgggtgggg gtccatcttt gggaccactg tcggcagagg catcttcaac gatggccttt 2580
   cctttatcgc aatgatggca tttgtaggag ccaccttcct tttccactat cttcacaata 2640
   aagtgacaga tagctgggca atggaatccg aggaggtttc cggatattac cctttgttga 2700
   aaagtctcca cccatgcaga tctgcaggca tgcaagctgc ggccgcgttt aaacaattcc 2760
   cgatcgatct agtaacatag atgacaccgc gcgcgataat ttatcctagt ttgcgcgcta 2820
   tattttgttt tctatcgcgt attaaatgta taattgcggg actctaatca taaaaaccca 2880
   tctcataaat aacgtcatgc attacatgtt aattattaca tgcttaacgt aattcaacag 2940
   aaattatatg ataatcatcg caagaccggc aacaggattc aatcttaaga aactttattg 3000
   ccaaatgttt gaacgatcgg ggaaattcga gctctccaat tccccaccga ggctgtagcc 3060
   gacgatggtg cgccaggaga gttgttgatt cattgtttgc ctccctgctg cggtttttca 3120
   ccgaagttca tgccagtcca gcgtttttgc agcagaaaag ccgccgactt cggtttgcgg 3180
   tcgcgagtga agatcccttt cttgttaccg ccaacgcgca atatgccttg cgaggtcgca 3240
   aaatcggcga aattccatac ctgttcaccg acgacggcgc tgacgcgatc aaagacgcgg 3300
   tgatacatat ccagccatgc acactgatac tcttcactcc acatgtcggt gtacattgag 3360
   tgcagcccgg ctaacgtatc cacgccgtat tcggtgatga taatcggctg atgcagtttc 3420
   tcctgccagg ccagaagttc tttttccagt accttctctg ccgtttccaa atcgccgctt 3480
   tggacatacc atccgtaata acggttcagg cacagcacat caaagagatc gctgatggta 3540
   tcggtgtgag cgtcgcagaa cattacattg acgcaggtga tcggacgcgt cgggtcgagt 3600
   ttacgcgttg cttccgccag tggcgaaata ttcccgtgca cttgcggacg ggtatccggt 3660
   tcgttggcaa tactccacat caccacgctt gggtggtttt tgtcacgcgc tatcagctct 3720
   ttaatcgcct gtaagtgcgc ttgctgagtt tccccgttga ctgcctcttc gctgtacagt 3780
   tctttcggct tgttgcccgc ttcgaaacca atgcctaaag agaggttaaa gccgacagca 3840
   gcagtttcat caatcaccac gatgccatgt tcatctgccc agtcgagcat ctcttcagcg 3900
   taagggtaat gcgaggtacg gtaggagttg gccccaatcc agtccattaa tgcgtggtcg 3960
   tgcaccatca gcacgttatc gaatcctttg ccacgtaagt ccgcatcttc atgacgacca 4020
   aagccagtaa agtagaacgg tttgtggtta atcaggaact gttcgccctt cactgccact 4080
   gaccggatgc cgacgcgaag cgggtagata tcacactctg tctggctttt ggctgtgacg 4140
   cacagttcat agagataacc ttcacccggt tgccagaggt gcggattcac cacttgcaaa 4200
   gtcccgctag tgccttgtcc agttgcaacc acctgttgat ccgcatcacg cagttcaacg 4260
   ctgacatcac cattggccac cacctgccag tcaacagacg cgtggttaca gtcttgcgcg 4320
   acatgcgtca ccacggtgat atcgtccacc caggtgttcg gcgtggtgta gagcattacg 4380
   ctgcgatgga ttccggcata gttaaagaaa tcatggaagt aagactgctt tttcttgccg 4440
   ttttcgtcgg taatcaccat tcccggcggg atagtctgcc agttcagttc gttgttcaca 4500
   caaacggtga tacgtacact tttcccggca ataacatacg gcgtgacatc ggcttcaaat 4560
   ggcgtatagc cgccctgatg ctccatcact tcctgattat tgacccacac tttgccgtaa 4620
   tgagtgaccg catcgaaacg cagcacgata cgctggcctg cccaaccttt cggtataaag 4680
   acttcgcgct gataccagac gttgcccgca taattacgaa tatctgcatc ggcgaactga 4740
   tcgttaaaac tgcctggcac agcaattgcc cggctttctt gtaacgcgct ttcccaccaa 4800
   cgctgatcaa ttccacagtt ttcgcgatcc agactgaatg cccacaggcc gtcgagtttt 4860
   ttgatttcac gggttggggt ttctacagga cggaccatgg aactttgctg gtgaaagtgg 4920
   caagctacac caagaactgc acaaaggaaa tgaatcataa gaacaagcag tataactatc 4980
   ccgattacag cttcataaat ttctttctgt aatcaagcag tcaaggaata accaaaaaga 5040
   aacttctaca ggtagataca agaggataat cgatagataa tcgatgtgaa tttgaaaaca 5100
   aaattgagga cagttcatgc cagactttca gctcagaata atgaaggggt gagagctaat 5160
   aaaaaaaata atccaaatga atactgctta tgctacagtt catttcatat aattaaaaat 5220
   ggagatcaga aatcggataa gttcttctcc tgactaaact ataggggtat tacctattaa 5280
   ctgaactaca gctaggataa cgctataaaa gcattatttg tagcaatcaa ttcattgaac 5340
   tgaaccagaa agtgactttt taagatattt gggaaaaaaa attctgggac taaagcaaat 5400
   cggaagaaca gggaatcccc tgttcctgag gattataact gtttccatga atttcgtgat 5460
   ggggactact gtatctaaac aagccagctg cgaaattttg gacaggctta agaatcaatc 5520
   attcaatctc atcaacggga ccgtcttcaa agttggatta tttttgttca atagggaata 5580
   aacaaaggat agcttcgtca aatcgagaag catcactacc agaatcgagg accaaacaac 5640
   cgtactttta ttacaccaag caaaatcacc ggtgctctga ttttacctca aacaaaaggt 5700
   actcacaaaa tcgcaagatt ccgtacccta aaccatttca tttccataga gctctccaga 5760
   cgattgaaaa ccatactact aatcaaaccg aaccgataac atgcaacatc aagaacccct 5820
   ctatcccaaa tccaagaaca ggaatcatca cagatacaga tcccctttcc taacatcaac 5880
   gcccgtacta cacaacctag aacaataaat ccaagaacaa ccgaagggca cataccctgt 5940
   gaggaggagg tggaggaggg aagagatcga ccaagaactc gaccggaaga tatggatcga 6000
   agaaggcggt cgctcggctt ctgctagtcg cgtgtccttg gtgctctccc tcttcttggc 6060
   ttggatgcct cctatataga gaggggaaaa ggggggagga atttatagat gggaggagga 6120
   ggatgcttgg ttttctttgc ctctcctcct ctctcctggc cgcaaagcct gctgttaaaa 6180
   ggttgttgcg gacttgctgt cctccgttag atcatcgttt tttgtgggtt gttctgtggg 6240
   cagccactct gttgttgcct gtgtgcccaa tatgggagat tggcgctcgc gcacgagcaa 6300
   aattcttttt ttttttgtat gtgaaaaacc taaatagttc gtttcatact tttcatgcta 6360
   ttctgtaaaa ttattttttg tattttagat attattaaaa gtggtctggt gatggtggag 6420
   tgcttgaatt cagatattgc tacctaaatg tattaaatag gcgagtgata ttagagatgt 6480
   gttgctagtt gaacgtgtat tgaggaggtg cactcacaca tgcacatgag cacaaagtgt 6540
   gtgcgtaagt accttgcatc taatcgaaaa aagataaata ataaaaataa gtcaattgtc 6600
   tttaattact aaataaaaat tgggatggag taagacatgt ccttaatgat gtgcgatatg 6660
   acgaatgcac aaactataaa attatataat tatatgttta aatctttgcc aatacgttca 6720
   gaatatcttc attctatttt tttaaaaaaa atctctctct ttacccattg agttcagcta 6780
   gaaagatttt tttattcaag aagagtttga tgacaattat ggctacctcg aataatttaa 6840
   cttcatgaca gagatgtgaa cgtatattct aagcaataat gatttcattt tcccacttaa 6900
   ttactaagga aaacgaaact agtgtagcga ctctttttta tttagcccga ttgccactaa 6960
   agtaggtgga tgagtttttc ttgcatagtt ctagttatca gcgctacata tataaggtct 7020
   cattttatat ttagcacacg ttccctatat tgttagttag gggtgaaaaa cggtgcagaa 7080
   actgcaggca tgcaagcttc ccgggaatgc ggccgcgcgc cgcagctggc gtaatcatgg 7140
   tcatagctgt ttcctgtgtg aaattgttat ccgctcacaa ttccacacaa catacgagcc 7200
   ggaagcataa agtgtaaagc ctggggtgcc taatgagtga gctaactcac attaattgcg 7260
   ttgcgctcac tgcccgcttt ccagtcggga aacctgtcgt gccagctgca ttaatgaatc 7320
   ggccaacgcg cggggagagg cggtttgcgt attgggcgct cttccgcttc ctcgctcact 7380
   gactcgctgc gctcggtcgt tcggctgcgg cgagcggtat cagctcactc aaaggcggta 7440
   atacggttat ccacagaatc aggggataac gcaggaaaga acatgtgagc aaaaggccag 7500
   caaaaggcca ggaaccgtaa aaaggccgcg ttgctggcgt ttttccatag gctccgcccc 7560
   cctgacgagc atcacaaaaa tcgacgctca agtcagaggt ggcgaaaccc gacaggacta 7620
   taaagatacc aggcgtttcc ccctggaagc tccctcgtgc gctctcctgt tccgaccctg 7680
   ccgcttaccg gatacctgtc cgcctttctc ccttcgggaa gcgtggcgct ttctcaatgc 7740
   tcacgctgta ggtatctcag ttcggtgtag gtcgttcgct ccaagctggg ctgtgtgcac 7800
   gaaccccccg ttcagcccga ccgctgcgcc ttatccggta actatcgtct tgagtccaac 7860
   ccggtaagac acgacttatc gccactggca gcagccactg gtaacaggat tagcagagcg 7920
   aggtatgtag gcggtgctac agagttcttg aagtggtggc ctaactacgg ctacactaga 7980
   aggacagtat ttggtatctg cgctctgctg aagccagtta ccttcggaaa aagagttggt 8040
   agctcttgat ccggcaaaca aaccaccgct ggtagcggtg gtttttttgt ttgcaagcag 8100
   cagattacgc gcagaaaaaa aggatctcaa gaagatcctt tgatcttttc tacggggtct 8160
   gacgctcagt ggaacgaaaa ctcacgttaa gggattttgg tcatgagatt atcaaaaagg 8220
   atcttcacct agatcctttt aaattaaaaa tgaagtttta aatcaatcta aagtatatat 8280
   gagtaaactt ggtctgacag ttaccaatgc ttaatcagtg aggcacctat ctcagcgatc 8340
   tgtctatttc gttcatccat agttgcctga ctccccgtcg tgtagataac tacgatacgg 8400
   gagggcttac catctggccc cagtgctgca atgataccgc gagacccacg ctcaccggct 8460
   ccagatttat cagcaataaa ccagccagcc ggaagggccg agcgcagaag tggtcctgca 8520
   actttatccg cctccatcca gtctattaat tgttgccggg aagctagagt aagtagttcg 8580
   ccagttaata gtttgcgcaa cgttgttgcc attgctacag gcatcgtggt gtcacgctcg 8640
   tcgtttggta tggcttcatt cagctccggt tcccaacgat caaggcgagt tacatgatcc 8700
   cccatgttgt gcaaaaaagc ggttagctcc ttcggtcctc cgatcgttgt cagaagtaag 8760
   ttggccgcag tgttatcact catggttatg gcagcactgc ataattctct tactgtcatg 8820
   ccatccgtaa gatgcttttc tgtgactggt gagtactcaa ccaagtcatt ctgagaatag 8880
   tgtatgcggc gaccgagttg ctcttgcccg gcgtcaatac gggataatac cgcgccacat 8940
   agcagaactt taaaagtgct catcattgga aaacgttctt cggggcgaaa actctcaagg 9000
   atcttaccgc tgttgagatc cagttcgatg taacccactc gtgcacccaa ctgatcttca 9060
   gcatctttta ctttcaccag cgtttctggg tgagcaaaaa caggaaggca aaatgccgca 9120
   aaaaagggaa taagggcgac acggaaatgt tgaatactca tactcttcct ttttcaatat 9180
   tattgaagca tttatcaggg ttattgtctc atgagcggat acatatttga atgtatttag 9240
   aaaaataaac aaataggggt tccgcgcaca tttccccgaa aagtgccacc tgacgtctaa 9300
   gaaaccatta ttatcatgac attaacctat aaaaataggc gtatcacgag gccctttcgt 9360
   c                                                                 9361
<210> 28
   <211> 10629
   <212> DNA
   <213> Artificial Sequence
<220>
   <223> Description of Artificial Sequence:pArGOS2Af-hpt
<400> 28
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt cccgatcgat ctagtaacat 420
   agatgacacc gcgcgcgata atttatccta gtttgcgcgc tatattttgt tttctatcgc 480
   gtattaaatg tataattgcg ggactctaat cataaaaacc catctcataa ataacgtcat 540
   gcattacatg ttaattatta catgcttaac gtaattcaac agaaattata tgataatcat 600
   cgcaagaccg gcaacaggat tcaatcttaa gaaactttat tgccaaatgt ttgaacgatc 660
   ggggaaattc gagctcttac tattcctttg ccctcggacg agtgctgggg cgtcggtttc 720
   cactatcggc gagtacttct acacagccat cggtccagac ggccgcgctt ctgcgggcga 780
   tttgtgtacg cccgacagtc ccggctccgg atcggacgat tgcgtcgcat cgaccctgcg 840
   cccaagctgc atcatcgaaa ttgccgtcaa ccaagctctg atagagttgg tcaagaccaa 900
   tgcggagcat atacgcccgg agccgcggcg atcctgcaag ctccggatgc ctccgctcga 960
   agtagcgcgt ctgctgctcc atacaagcca accacggcct ccagaagaag atgttggcga 1020
   cctcgtattg ggaatccccg aacatcgcct cgctccagtc aatgaccgct gttatgcggc 1080
   cattgtccgt caggacattg ttggagccga aatccgcgtg cacgaggtgc cggacttcgg 1140
   ggcagtcctc ggcccaaagc atcagctcat cgagagcctg cgcgacggac gcactgacgg 1200
   tgtcgtccat cacagtttgc cagtgataca catggggatc agcaatcgcg catatgaaat 1260
   cacgccatgt agtgtattga ccgattcctt gcggtccgaa tgggccgaac ccgctcgtct 1320
   ggctaagatc ggccgcagcg atcgcatcca tggcctccgc gaccggctgc agaacagcgg 1380
   gcagttcggt ttcaggcagg tcttgcaacg tgacaccctg tgcacggcgg gagatgcaat 1440
   aggtcaggct ctcgctgaat tccccaatgt caagcacttc cggaatcggg agcgcggccg 1500
   atgcaaagtg ccgataaaca taacgatctt tgtagaaacc atcggcgcag ctatttaccc 1560
   gcaggacata tccacgccct cctacatcga agctgaaagc acgagattct tcgccctccg 1620
   agagctgcat caggtcggag acgctgtcga acttttcgat cagaaacttc tcgacagacg 1680
   tcgcggtgag ttcaggcttt ttcatatctc acgagatctg tatcccgtgc ctggaacaaa 1740
   tggccccaga tccgtccgca gctgcacggg tccaggaaag caatcgcata gtcaagctaa 1800
   atcatcaaga tgcaaacttt tcgcccttgc taaacacggt aaaattcgaa tggacatgtg 1860
   tggagcagca aaggagcttt ccccaaaatt actcaacgaa tcataaacca agattagtca 1920
   gatcaagaga cagaggagaa acaaggcgga cctttgcact tgatctgggg attgccctga 1980
   cttggtggtg ctggtatatt agggataggg ttgctcctat ccacagcttg tccaccaaat 2040
   atcagctcct ccgtggactg ccttgtcgag ccttcagctg gatcctctag agtcccccgt 2100
   gttctctcca aatgaaatga acttccttat atagaggaag ggtcttgcga aggatagtgg 2160
   gattgtgcgt catcccttac gtcagtggag atatcacatc aatccacttg ctttgaagac 2220
   gtggttggaa cgtcttcttt ttccacgatg ctcctcgtgg gtgggggtcc atctttggga 2280
   ccactgtcgg cagaggcatc ttcaacgatg gcctttcctt tatcgcaatg atggcatttg 2340
   taggagccac cttccttttc cactatcttc acaataaagt gacagatagc tgggcaatgg 2400
   aatccgagga ggtttccgga tattaccctt tgttgaaaag tctccatcga tgatcacatc 2460
   aatccacttg ctttgaagac gtggttggaa cgtcttcttt ttccacgatg ctcctcgtgg 2520
   gtgggggtcc atctttggga ccactgtcgg cagaggcatc ttcaacgatg gcctttcctt 2580
   tatcgcaatg atggcatttg taggagccac cttccttttc cactatcttc acaataaagt 2640
   gacagatagc tgggcaatgg aatccgagga ggtttccgga tattaccctt tgttgaaaag 2700
   tctccaccca tgcagatctg caggcatgca agctgcggcc gctctagaac tagtggatcc 2760
   tgcaagagtt ttacaagtga aattaagttt aaacatttat aaatatatac caagtcaagt 2820
   gtgcttttta aaaattttgc tgacttcata aaatatatta ggaagtcagc ttggcatgga 2880
   gctatataat cttaaaaagg aatcaaaaat atcgaaaaat atattagaag ggagaggact 2940
   aattaatatt taccagctcc agggaaatat attctttgga gctggcctat cgttttaaaa 3000
   ttttttttca tggacgtcat atataaatct aaaccccttc taaatatatt agaaattaaa 3060
   gatatcgaga tcctgcaaga gttttacaag tgaaattaag tttaaacatt tataaatata 3120
   taccaagtca agtgtgcttt ttaaaaattt tgctgacttc ataaaatata ttaggaagtc 3180
   agcttggcat ggagctatat aatcttaaaa aggaatcaaa aatatcgaaa aatatattag 3240
   aagggagagg actaattaat atttaccagc tccagggaaa tatattcttt ggagctggcc 3300
   tatcgtttta aaattttttt tcatggacgt catatataaa tctaaacccc ttctaaatat 3360
   attagaaatt aaagatatcg agatccccca aacaattccc gatcgatcta gtaacataga 3420
   tgacaccgcg cgcgataatt tatcctagtt tgcgcgctat attttgtttt ctatcgcgta 3480
   ttaaatgtat aattgcggga ctctaatcat aaaaacccat ctcataaata acgtcatgca 3540
   ttacatgtta attattacat gcttaacgta attcaacaga aattatatga taatcatcgc 3600
   aagaccggca acaggattca atcttaagaa actttattgc caaatgtttg aacgatcggg 3660
   gaaattcgag ctctccaatt ccccaccgag gctgtagccg acgatggtgc gccaggagag 3720
   ttgttgattc attgtttgcc tccctgctgc ggtttttcac cgaagttcat gccagtccag 3780
   cgtttttgca gcagaaaagc cgccgacttc ggtttgcggt cgcgagtgaa gatccctttc 3840
   ttgttaccgc caacgcgcaa tatgccttgc gaggtcgcaa aatcggcgaa attccatacc 3900
   tgttcaccga cgacggcgct gacgcgatca aagacgcggt gatacatatc cagccatgca 3960
   cactgatact cttcactcca catgtcggtg tacattgagt gcagcccggc taacgtatcc 4020
   acgccgtatt cggtgatgat aatcggctga tgcagtttct cctgccaggc cagaagttct 4080
   ttttccagta ccttctctgc cgtttccaaa tcgccgcttt ggacatacca tccgtaataa 4140
   cggttcaggc acagcacatc aaagagatcg ctgatggtat cggtgtgagc gtcgcagaac 4200
   attacattga cgcaggtgat cggacgcgtc gggtcgagtt tacgcgttgc ttccgccagt 4260
   ggcgaaatat tcccgtgcac ttgcggacgg gtatccggtt cgttggcaat actccacatc 4320
   accacgcttg ggtggttttt gtcacgcgct atcagctctt taatcgcctg taagtgcgct 4380
   tgctgagttt ccccgttgac tgcctcttcg ctgtacagtt ctttcggctt gttgcccgct 4440
   tcgaaaccaa tgcctaaaga gaggttaaag ccgacagcag cagtttcatc aatcaccacg 4500
   atgccatgtt catctgccca gtcgagcatc tcttcagcgt aagggtaatg cgaggtacgg 4560
   taggagttgg ccccaatcca gtccattaat gcgtggtcgt gcaccatcag cacgttatcg 4620
   aatcctttgc cacgtaagtc cgcatcttca tgacgaccaa agccagtaaa gtagaacggt 4680
   ttgtggttaa tcaggaactg ttcgcccttc actgccactg accggatgcc gacgcgaagc 4740
   gggtagatat cacactctgt ctggcttttg gctgtgacgc acagttcata gagataacct 4800
   tcacccggtt gccagaggtg cggattcacc acttgcaaag tcccgctagt gccttgtcca 4860
   gttgcaacca cctgttgatc cgcatcacgc agttcaacgc tgacatcacc attggccacc 4920
   acctgccagt caacagacgc gtggttacag tcttgcgcga catgcgtcac cacggtgata 4980
   tcgtccaccc aggtgttcgg cgtggtgtag agcattacgc tgcgatggat tccggcatag 5040
   ttaaagaaat catggaagta agactgcttt ttcttgccgt tttcgtcggt aatcaccatt 5100
   cccggcggga tagtctgcca gttcagttcg ttgttcacac aaacggtgat acgtacactt 5160
   ttcccggcaa taacatacgg cgtgacatcg gcttcaaatg gcgtatagcc gccctgatgc 5220
   tccatcactt cctgattatt gacccacact ttgccgtaat gagtgaccgc atcgaaacgc 5280
   agcacgatac gctggcctgc ccaacctttc ggtataaaga cttcgcgctg ataccagacg 5340
   ttgcccgcat aattacgaat atctgcatcg gcgaactgat cgttaaaact gcctggcaca 5400
   gcaattgccc ggctttcttg taacgcgctt tcccaccaac gctgatcaat tccacagttt 5460
   tcgcgatcca gactgaatgc ccacaggccg tcgagttttt tgatttcacg ggttggggtt 5520
   tctacaggac ggaccatgga actttgctgg tgaaagtggc aagctacacc aagaactgca 5580
   caaaggaaat gaatcataag aacaagcagt ataactatcc cgattacagc ttcataaatt 5640
   tctttctgta atcaagcagt caaggaataa ccaaaaagaa acttctacag gtagatacaa 5700
   gaggataatc gatagataat cgatgtgaat ttgaaaacaa aattgaggac agttcatgcc 5760
   agactttcag ctcagaataa tgaaggggtg agagctaata aaaaaaataa tccaaatgaa 5820
   tactgcttat gctacagttc atttcatata attaaaaatg gagatcagaa atcggataag 5880
   ttcttctcct gactaaacta taggggtatt acctattaac tgaactacag ctaggataac 5940
   gctataaaag cattatttgt agcaatcaat tcattgaact gaaccagaaa gtgacttttt 6000
   aagatatttg ggaaaaaaaa ttctgggact aaagcaaatc ggaagaacag ggaatcccct 6060
   gttcctgagg attataactg tttccatgaa tttcgtgatg gggactactg tatctaaaca 6120
   agccagctgc gaaattttgg acaggcttaa gaatcaatca ttcaatctca tcaacgggac 6180
   cgtcttcaaa gttggattat ttttgttcaa tagggaataa acaaaggata gcttcgtcaa 6240
   atcgagaagc atcactacca gaatcgagga ccaaacaacc gtacttttat tacaccaagc 6300
   aaaatcaccg gtgctctgat tttacctcaa acaaaaggta ctcacaaaat cgcaagattc 6360
   cgtaccctaa accatttcat ttccatagag ctctccagac gattgaaaac catactacta 6420
   atcaaaccga accgataaca tgcaacatca agaacccctc tatcccaaat ccaagaacag 6480
   gaatcatcac agatacagat cccctttcct aacatcaacg cccgtactac acaacctaga 6540
   acaataaatc caagaacaac cgaagggcac ataccctgtg aggaggaggt ggaggaggga 6600
   agagatcgac caagaactcg accggaagat atggatcgaa gaaggcggtc gctcggcttc 6660
   tgctagtcgc gtgtccttgg tgctctccct cttcttggct tggatgcctc ctatatagag 6720
   aggggaaaag gggggaggaa tttatagatg ggaggaggag gatgcttggt tttctttgcc 6780
   tctcctcctc tctcctggcc gcaaagcctg ctgttaaaag gttgttgcgg acttgctgtc 6840
   ctccgttaga tcatcgtttt ttgtgggttg ttctgtgggc agccactctg ttgttgcctg 6900
   tgtgcccaat atgggagatt ggcgctcgcg cacgagcaaa attctttttt tttttgtatg 6960
   tgaaaaacct aaatagttcg tttcatactt ttcatgctat tctgtaaaat tattttttgt 7020
   attttagata ttattaaaag tggtctggtg atggtggagt gcttgaattc agatattgct 7080
   acctaaatgt attaaatagg cgagtgatat tagagatgtg ttgctagttg aacgtgtatt 7140
   gaggaggtgc actcacacat gcacatgagc acaaagtgtg tgcgtaagta ccttgcatct 7200
   aatcgaaaaa agataaataa taaaaataag tcaattgtct ttaattacta aataaaaatt 7260
   gggatggagt aagacatgtc cttaatgatg tgcgatatga cgaatgcaca aactataaaa 7320
   ttatataatt atatgtttaa atctttgcca atacgttcag aatatcttca ttctattttt 7380
   ttaaaaaaaa tctctctctt tacccattga gttcagctag aaagattttt ttattcaaga 7440
   agagtttgat gacaattatg gctacctcga ataatttaac ttcatgacag agatgtgaac 7500
   gtatattcta agcaataatg atttcatttt cccacttaat tactaaggaa aacgaaacta 7560
   gtgtagcgac tcttttttat ttagcccgat tgccactaaa gtaggtggat gagtttttct 7620
   tgcatagttc tagttatcag cgctacatat ataaggtctc attttatatt tagcacacgt 7680
   tccctatatt gttagttagg gggtgaaaac ggtgcagaaa ctgcagcccg ggggatctcg 7740
   atatctttaa tttctaatat atttagaagg ggtttagatt tatatatgac gtccatgaaa 7800
   aaaaatttta aaacgatagg ccagctccaa agaatatatt tccctggagc tggtaaatat 7860
   taattagtcc tctcccttct aatatatttt tcgatatttt tgattccttt ttaagattat 7920
   atagctccat gccaagctga cttcctaata tattttatga agtcagcaaa atttttaaaa 7980
   agcacacttg acttggtata tatttataaa tgtttaaact taatttcact tgtaaaactc 8040
   ttgcaggatc tcgatatctt taatttctaa tatatttaga aggggtttag atttatatat 8100
   gacgtccatg aaaaaaaatt ttaaaacgat aggccagctc caaagaatat atttccctgg 8160
   agctggtaaa tattaattag tcctctccct tctaatatat ttttcgatat ttttgattcc 8220
   tttttaagat tatatagctc catgccaagc tgacttccta atatatttta tgaagtcagc 8280
   aaaattttta aaaagcacac ttgacttggt atatatttat aaatgtttaa acttaatttc 8340
   acttgtaaaa ctcttgcagg atccactagt tctagagcgg ccgcgcgccg cagctggcgt 8400
   aatcatggtc atagctgttt cctgtgtgaa attgttatcc gctcacaatt ccacacaaca 8460
   tacgagccgg aagcataaag tgtaaagcct ggggtgccta atgagtgagc taactcacat 8520
   taattgcgtt gcgctcactg cccgctttcc agtcgggaaa cctgtcgtgc cagctgcatt 8580
   aatgaatcgg ccaacgcgcg gggagaggcg gtttgcgtat tgggcgctct tccgcttcct 8640
   cgctcactga ctcgctgcgc tcggtcgttc ggctgcggcg agcggtatca gctcactcaa 8700
   aggcggtaat acggttatcc acagaatcag gggataacgc aggaaagaac atgtgagcaa 8760
   aaggccagca aaaggccagg aaccgtaaaa aggccgcgtt gctggcgttt ttccataggc 8820
   tccgcccccc tgacgagcat cacaaaaatc gacgctcaag tcagaggtgg cgaaacccga 8880
   caggactata aagataccag gcgtttcccc ctggaagctc cctcgtgcgc tctcctgttc 8940
   cgaccctgcc gcttaccgga tacctgtccg cctttctccc ttcgggaagc gtggcgcttt 9000
   ctcaatgctc acgctgtagg tatctcagtt cggtgtaggt cgttcgctcc aagctgggct 9060
   gtgtgcacga accccccgtt cagcccgacc gctgcgcctt atccggtaac tatcgtcttg 9120
   agtccaaccc ggtaagacac gacttatcgc cactggcagc agccactggt aacaggatta 9180
   gcagagcgag gtatgtaggc ggtgctacag agttcttgaa gtggtggcct aactacggct 9240
   acactagaag gacagtattt ggtatctgcg ctctgctgaa gccagttacc ttcggaaaaa 9300
   gagttggtag ctcttgatcc ggcaaacaaa ccaccgctgg tagcggtggt ttttttgttt 9360
   gcaagcagca gattacgcgc agaaaaaaag gatctcaaga agatcctttg atcttttcta 9420
   cggggtctga cgctcagtgg aacgaaaact cacgttaagg gattttggtc atgagattat 9480
   caaaaaggat cttcacctag atccttttaa attaaaaatg aagttttaaa tcaatctaaa 9540
   gtatatatga gtaaacttgg tctgacagtt accaatgctt aatcagtgag gcacctatct 9600
   cagcgatctg tctatttcgt tcatccatag ttgcctgact ccccgtcgtg tagataacta 9660
   cgatacggga gggcttacca tctggcccca gtgctgcaat gataccgcga gacccacgct 9720
   caccggctcc agatttatca gcaataaacc agccagccgg aagggccgag cgcagaagtg 9780
   gtcctgcaac tttatccgcc tccatccagt ctattaattg ttgccgggaa gctagagtaa 9840
   gtagttcgcc agttaatagt ttgcgcaacg ttgttgccat tgctacaggc atcgtggtgt 9900
   cacgctcgtc gtttggtatg gcttcattca gctccggttc ccaacgatca aggcgagtta 9960
   catgatcccc catgttgtgc aaaaaagcgg ttagctcctt cggtcctccg atcgttgtca 10020
   gaagtaagtt ggccgcagtg ttatcactca tggttatggc agcactgcat aattctctta 10080
   ctgtcatgcc atccgtaaga tgcttttctg tgactggtga gtactcaacc aagtcattct 10140
   gagaatagtg tatgcggcga ccgagttgct cttgcccggc gtcaatacgg gataataccg 10200
   cgccacatag cagaacttta aaagtgctca tcattggaaa acgttcttcg gggcgaaaac 10260
   tctcaaggat cttaccgctg ttgagatcca gttcgatgta acccactcgt gcacccaact 10320
   gatcttcagc atcttttact ttcaccagcg tttctgggtg agcaaaaaca ggaaggcaaa 10380
   atgccgcaaa aaagggaata agggcgacac ggaaatgttg aatactcata ctcttccttt 10440
   ttcaatatta ttgaagcatt tatcagggtt attgtctcat gagcggatac atatttgaat 10500
   gtatttagaa aaataaacaa ataggggttc cgcgcacatt tccccgaaaa gtgccacctg 10560
   acgtctaaga aaccattatt atcatgacat taacctataa aaataggcgt atcacgaggc 10620
   cctttcgtc                                                         10629
<210> 29
   <211> 676
   <212> DNA
   <213> Artificial Sequence
<220>
   <223> Description of Artificial Sequence: 5' MAR in Ar-Act-Af
<400> 29
   ctccaccgcg gtggcggccg ctctagaact agtggatcct gcaagagttt tacaagtgaa 60
   attaagttta aacatttata aatatatacc aagtcaagtg tgctttttaa aaattttgct 120
   gacttcataa aatatattag gaagtcagct tggcatggag ctatataatc ttaaaaagga 180
   atcaaaaata tcgaaaaata tattagaagg gagaggacta attaatattt accagctcca 240
   gggaaatata ttctttggag ctggcctatc gttttaaaat tttttttcat ggacgtcata 300
   tataaatcta aaccccttct aaatatatta gaaattaaag atatcgagat cctgcaagag 360
   ttttacaagt gaaattaagt ttaaacattt ataaatatat accaagtcaa gtgtgctttt 420
   taaaaatttt gctgacttca taaaatatat taggaagtca gcttggcatg gagctatata 480
   atcttaaaaa ggaatcaaaa atatcgaaaa atatattaga agggagagga ctaattaata 540
   tttaccagct ccagggaaat atattctttg gagctggcct atcgttttaa aatttttttt 600
   catggacgtc atatataaat ctaaacccct tctaaatata ttagaaatta aagatatcga 660
   gatcccccgg gctgca                                                 676
<210> 30
   <211> 15676
   <212> DNA
   <213> Artificial Sequence
<220>
   <223> Description of Artificial Sequence:pAct2-bin
<400> 30
   gatctgcaag cgatcgcggc gcgccattta aatgcccggg cgtttaaacg cggccgcatt 60
   cccgggaagc ttgcatgcct gcagaattcg gcttgttttg acgagttcgg atgtagtagt 120
   agccattatt taatgtacat actaatcgtg aatagtgaat atgatgaaac attgtatctt 180
   attgtataaa tatccataaa cacatcatga aagacacttt ctttcacggt ctgaattaat 240
   tatgatacaa ttctaataga aaacgaatta aattacgttg aattgtatga aatctaattg 300
   aacaagccaa ccacgacgac gactaacgtt gcctggattg actcggttta agttaaccac 360
   taaaaaaacg gagctgtcat gtaacacgcg gatcgagcag gtcacagtca tgaagccatc 420
   aaagcaaaag aactaatcca aggggctgag atgattaatt agtttaaaaa ttagttaaca 480
   cgagggaaaa ggctgcctga cagccaggtc acgttatctt tacctgtggt cgaaatgatt 540
   cgtgtctgtc gattttaatt atttttttga aaggccgaaa ataaagttgt aagagataaa 600
   cccgcctata taaattcata tattttcctc tccgctttga attgtctcgt tgtcctcctc 660
   actttcatca gccgttttga atctccggcg acttgacaga gaagaacaag gaagaagact 720
   aagagagaaa gtaagagata atccaggaga ttcattctcc gttttgaatc ttcctcaatc 780
   tcatcttctt ccgctctttc tttccaaggt aataggaact ttctggatct actttatttg 840
   ctggatctcg atcttgtttt ctcaatttcc ttgagatctg gaattcgttt aatttgggat 900
   ctgtgaacct ccactaaatc ttttggtttt actagaatcg atctaagttg accgatcagt 960
   tagctcgatt atagctacca gaatttggct tgaccttgat ggagagatcc atgttcatgt 1020
   tacctgggaa atgatttgta tatgtgaaat gaaatctgaa ctgttgaagt tagattgaat 1080
   ctgaacactg tcaatgttag attgaatctg aacactgttt aaggttagat gaagtttgtg 1140
   tatagattct tcgaaacttt aggatttgta gtgtcgtacg ttgaacagaa agctatttct 1200
   gattcaatca gggtttattt gactgtattg aactcttttt gtgtgtttgc agctgtagac 1260
   catggtccgt cctgtagaaa ccccaacccg tgaaatcaaa aaactcgacg gcctgtgggc 1320
   attcagtctg gatcgcgaaa actgtggaat tgatcagcgt tggtgggaaa gcgcgttaca 1380
   agaaagccgg gcaattgctg tgccaggcag ttttaacgat cagttcgccg atgcagatat 1440
   tcgtaattat gcgggcaacg tctggtatca gcgcgaagtc tttataccga aaggttgggc 1500
   aggccagcgt atcgtgctgc gtttcgatgc ggtcactcat tacggcaaag tgtgggtcaa 1560
   taatcaggaa gtgatggagc atcagggcgg ctatacgcca tttgaagccg atgtcacgcc 1620
   gtatgttatt gccgggaaaa gtgtacgtat caccgtttgt gtgaacaacg aactgaactg 1680
   gcagactatc ccgccgggaa tggtgattac cgacgaaaac ggcaagaaaa agcagtctta 1740
   cttccatgat ttctttaact atgccggaat ccatcgcagc gtaatgctct acaccacgcc 1800
   gaacacctgg gtggacgata tcaccgtggt gacgcatgtc gcgcaagact gtaaccacgc 1860
   gtctgttgac tggcaggtgg tggccaatgg tgatgtcagc gttgaactgc gtgatgcgga 1920
   tcaacaggtg gttgcaactg gacaaggcac tagcgggact ttgcaagtgg tgaatccgca 1980
   cctctggcaa ccgggtgaag gttatctcta tgaactgtgc gtcacagcca aaagccagac 2040
   agagtgtgat atctacccgc ttcgcgtcgg catccggtca gtggcagtga agggcgaaca 2100
   gttcctgatt aaccacaaac cgttctactt tactggcttt ggtcgtcatg aagatgcgga 2160
   cttacgtggc aaaggattcg ataacgtgct gatggtgcac gaccacgcat taatggactg 2220
   gattggggcc aactcctacc gtacctcgca ttacccttac gctgaagaga tgctcgactg 2280
   ggcagatgaa catggcatcg tggtgattga tgaaactgct gctgtcggct ttaacctctc 2340
   tttaggcatt ggtttcgaag cgggcaacaa gccgaaagaa ctgtacagcg aagaggcagt 2400
   caacggggaa actcagcaag cgcacttaca ggcgattaaa gagctgatag cgcgtgacaa 2460
   aaaccaccca agcgtggtga tgtggagtat tgccaacgaa ccggataccc gtccgcaagt 2520
   gcacgggaat atttcgccac tggcggaagc aacgcgtaaa ctcgacccga cgcgtccgat 2580
   cacctgcgtc aatgtaatgt tctgcgacgc tcacaccgat accatcagcg atctctttga 2640
   tgtgctgtgc ctgaaccgtt attacggatg gtatgtccaa agcggcgatt tggaaacggc 2700
   agagaaggta ctggaaaaag aacttctggc ctggcaggag aaactgcatc agccgattat 2760
   catcaccgaa tacggcgtgg atacgttagc cgggctgcac tcaatgtaca ccgacatgtg 2820
   gagtgaagag tatcagtgtg catggctgga tatgtatcac cgcgtctttg atcgcgtcag 2880
   cgccgtcgtc ggtgaacagg tatggaattt cgccgatttt gcgacctcgc aaggcatatt 2940
   gcgcgttggc ggtaacaaga aagggatctt cactcgcgac cgcaaaccga agtcggcggc 3000
   ttttctgctg caaaaacgct ggactggcat gaacttcggt gaaaaaccgc agcagggagg 3060
   caaacaatga atcaacaact ctcctggcgc accatcgtcg gctacagcct cggtggggaa 3120
   ttggagagct cgaatttccc cgatcgttca aacatttggc aataaagttt cttaagattg 3180
   aatcctgttg ccggtcttgc gatgattatc atataatttc tgttgaatta cgttaagcat 3240
   gtaataatta acatgtaatg catgacgtta tttatgagat gggtttttat gattagagtc 3300
   ccgcaattat acatttaata cgcgatagaa aacaaaatat agcgcgcaaa ctaggataaa 3360
   ttatcgcgcg cggtgtcatc tatgttacta gatcgatcgg gaattgttta aacgcggccg 3420
   cttaattaag gccggcctgc aggcatgcaa gctcggatct cacctacgat ggggggcatc 3480
   gcaccggtga gtaatattgt acggctaaga gcgaatttgg cctgtagacc tcaattgcga 3540
   gctttctaat ttcaaactat tcgggcctaa cttttggtgt gatgatgctg actggcagga 3600
   tatataccgt tgtaatttga gctcgtgtga ataagtcgct gtgtatgttt gtttgattgt 3660
   ttctgttgga gtgcagccca tttcaccgga caagtcggct agattgattt agccctgatg 3720
   aactgccgag ggaagccatt cttgagcgcg gaatgggaat ggatttcgtt gtacaacgag 3780
   acgacagaac acccacggga ccgagcttcg caagcttgtt gtaactgaaa aaggaaaatt 3840
   attgtgccag gcagttgaaa gtcagcacct tttaacgagt gctgaaatga cggctaaatg 3900
   ggaaacgtat ttaaaaaaaa tcggtaaaag agaaggcaat caagagaact ttattacgaa 3960
   tatcaaaaaa ttcattgttc atttactgga agctgtacct aacgatatag aaaaactaaa 4020
   tttttctgat taccaggaac agaaagaaaa agaagcagaa aaaagtattg taggaaaatg 4080
   tcctaagtgt ggcaacaata ttgtattaaa aaaatcgttt tatggttgtt caaattatcc 4140
   tgaatgtaag tttactttag ctgaacattt tagaaagaaa aaactcacca aaacaaatgt 4200
   aaaagaatta ctagagggaa aagaaaccct ggtaaaagga atcaaaacga aagatagaaa 4260
   gtcctacaat gccgttgtaa aaatcggaga aaagggatat attgatttta tatctttctc 4320
   aaaataaaca taaaagccct ttaaagaggg cttttatata ttaatcacaa atcacttatc 4380
   acaaatcaca agtgatttgt gattgttgat gataaaataa gaataagaag aaatagaaag 4440
   aagtgagtga ttgtgggaaa tttaggcgca caaaaagaaa aacgaaatga tacaccaatc 4500
   agtgcaaaaa aagatataat gggagataag acggttcgtg ttcgtgctga cttgcaccat 4560
   atcataaaaa tcgaaacagc aaagaatggc ggaaacgtaa aagaagttat ggaaataaga 4620
   cttagaagca aacttaagag tgtgttgata gtgcagtatc ttaaaatttt gtataatagg 4680
   aattgaagtt aaattagatg ctaaaaattt gtaattaaga aggagtgatt acatgaacaa 4740
   aaatataaaa tattctcaaa actttttaac gaatgaaaaa gtactcaacc aaataataaa 4800
   acaattgaat ttaaaagaaa ccgataccgt ttacgaaatt ggaacaggta aagggcattt 4860
   aacgacgaaa ctggctaaaa taagtaaaca ggtaacgtct attgaattag acagtcatct 4920
   attcaactta tcgtcagaaa aattaaaact gaatactcgt gtcactttaa ttcaccaaga 4980
   tattctacag tttcaattcc ctaacaaaca gaggtataaa attgttggga gtattcctta 5040
   ccatttaagc acacaaatta ttaaaaaagt ggtttttgaa agccatgcgt ctgacatcta 5100
   tctgattgtt gaaaaaaaaa aaaaaaagcg taccttggat attcaccgaa cactagggtt 5160
   gctcttgcac actcaagtct cgattcagca attgcttaag ctgccagcgg aatgctttca 5220
   tcctaaacca aaagtaaaca gtgtcttaat aaaacttacc cgccatacca cagatgttcc 5280
   agataaatat tggaagctat atacgtactt tgtttcaaaa tgggtcaatc gagaatatcg 5340
   tcaactgttt actaaaaatc agtttcatca agcaatgaaa cacgccaaag taaacaattt 5400
   aagtaccgtt acttatgagc aagtattgtc tatttttaat agttatctat tatttaacgg 5460
   gaggaaataa gggctgagaa agcccagtaa ggaaacaact gtaggttcga gtcgcgagat 5520
   cccccggaac caaaggaagt aggttaaacc cgctccgatc aggccgagcc acgccaggcc 5580
   gagaacattg gttccttgta ggcatcggga ttggcggatc aaacactaaa gctactggaa 5640
   cgagcagaag tcctccggcc gccagttgcc aggccgtaaa ggtgagcaga ggcacgggag 5700
   gttgccactt gcgggtcagc acggttccga acgccatgga aaccgccccc gccaggcccg 5760
   ctgcgacgcc gacaggatct agcgctgcgt ttggtgtcaa caccaacagc gccacgcccg 5820
   cagttccgca aatagccccc aggaccgcca tcaatcgtat cgggctacct agcagagcgg 5880
   cagagatgaa cacgaccatc agcggctgca cagcgcctac cgtcgccgcg acccgcccgg 5940
   caggcggtag accgaaataa acaacaagct ccagaatagc gaaatattaa gtgcgccgag 6000
   gatgaagatg cgcatccacc agattcccgt tggaatctgt cggacgatca tcacgagcaa 6060
   taaacccgcc ggcaacgccc gcagcagcat accggcgacc cctcggcctc gctgttcggg 6120
   ctccacgaaa acgccggaca gatgcgcctt gtgagcgtcc ttggggccgt cctcctgttt 6180
   gaagaccgac agcccaatga tctcgccgtc gatgtaggcg ccgaatgcca cggcatctcg 6240
   caaccgttca gcgaacgcct ccatgggctt tttctcctcg tgctcgtaaa cggacccgaa 6300
   catctctgga gctttcttca gggccgacaa tcggatctcg cggaaatcct gcacgtcggc 6360
   cgctccaagc cgtcgaatct gagccttaat cacaattgtc aattttaatc ctctgtttat 6420
   cggcagttcg tagagcgcgc cgtgcgcccg agcgatactg agcgaagcaa gtgcgtcgag 6480
   cagtgcccgc ttgttcctga aatgccagta aagcgctggc tgctgaaccc ccagccggaa 6540
   ctgaccccac aaggccctag cgtttgcaat gcaccaggtc atcattgacc caggcgtgtt 6600
   ccaccaggcc gctgcctcgc aactcttcgc aggcttcgcc gacctgctcg cgccacttct 6660
   tcacgcgggt ggaatccgat ccgcacatga ggcggaaggt ttccagcttg agcgggtacg 6720
   gctcccggtg cgagctgaaa tagtcgaaca tccgtcgggc cgtcggcgac agcttgcggt 6780
   acttctccca tatgaatttc gtgtagtggt cgccagcaaa cagcacgacg atttcctcgt 6840
   cgatcaggac ctggcaacgg gacgttttct tgccacggtc caggacgcgg aagcggtgca 6900
   gcagcgacac cgattccagg tgcccaacgc ggtcggacgt gaagcccatc gccgtcgcct 6960
   gtaggcgcga caggcattcc tcggccttcg tgtaataccg gccattgatc gaccagccca 7020
   ggtcctggca aagctcgtag aacgtgaagg tgatcggctc gccgataggg gtgcgcttcg 7080
   cgtactccaa cacctgctgc cacaccagtt cgtcatcgtc ggcccgcagc tcgacgccgg 7140
   tgtaggtgat cttcacgtcc ttgttgacgt ggaaaatgac cttgttttgc agcgcctcgc 7200
   gcgggatttt cttgttgcgc gtggtgaaca gggcagagcg ggccgtgtcg tttggcatcg 7260
   ctcgcatcgt gtccggccac ggcgcaatat cgaacaagga aagctgcatt tccttgatct 7320
   gctgcttcgt gtgtttcagc aacgcggcct gcttggcctc gctgacctgt tttgccaggt 7380
   cctcgccggc ggtttttcgc ttcttggtcg tcatagttcc tcgcgtgtcg atggtcatcg 7440
   acttcgccaa acctgccgcc tcctgttcga gacgacgcga acgctccacg gcggccgatg 7500
   gcgcgggcag ggcaggggga gccagttgca cgctgtcgcg ctcgatcttg gccgtagctt 7560
   gctggaccat cgagccgacg gactggaagg tttcgcgggg cgcacgcatg acggtgcggc 7620
   ttgcgatggt ttcggcatcc tcggcggaaa accccgcgtc gatcagttct tgcctgtatg 7680
   ccttccggtc aaacgtccga ttcattcacc ctccttgcgg gattgccccg actcacgccg 7740
   gggcaatgtg cccttattcc tgatttgacc cgcctggtgc cttggtgtcc agataatcca 7800
   ccttatcggc aatgaagtcg gtcccgtaga ccgtctggcc gtccttctcg tacttggtat 7860
   tccgaatctt gccctgcacg aataccagcg accccttgcc caaatacttg ccgtgggcct 7920
   cggcctgaga gccaaaacac ttgatgcgga agaagtcggt gcgctcctgc ttgtcgccgg 7980
   catcgttgcg ccactcttca ttaaccgcta tatcgaaaat tgcttgcggc ttgttagaat 8040
   tgccatgacg tacctcggtg tcacgggtaa gattaccgat aaactggaac tgattatggc 8100
   nnctcgaaat tccctcggtc ttgccttgct cgtcggtgat gtacttcacc agctccgcga 8160
   agtcgctctt cttgatggag cgcatgggga cgtgcttggc aatcacgcgc accccccggc 8220
   cgttttagcg gctaaaaaag tcatggctct gccctcgggc ggaccacgcc catcatgacc 8280
   ttgccaagct cgtcctgctt ctcttcgatc ttcgccagca gggcgaggat cgtggcatca 8340
   ccgaaccgcg ccgtgcgcgg gtcgtcggtg agccagagtt tcagcaggcc gcccaggcgg 8400
   cccaggtcgc cattgatgcg ggccagctcg cggacgtgct catagtccac gacgcccgtg 8460
   attttgtagc cctggccgac ggccagcagg taggccgaca ggctcatgcc ggccgccgcc 8520
   gccttttcct caatcgctct tcgttcgtct ggaaggcagt acaccttgat aggtgggctg 8580
   cccttcctgg ttggcttggt ttcatcagcc atccgcttgc cctcatctgt tacgccggcg 8640
   gtagccggcc agcctcgcag agcaggattc ccgttgagca ccgccaggtg cgaataaggg 8700
   acagtgaaga aggaacaccc gctcgcgggt gggcctactt cacctatcct gcccggctga 8760
   cgccgttgga tacaccaagg aaagtctaca cgaacccttt ggcaaaatcc tgtatatcgt 8820
   gcgaaaaagg atggatatac cgaaaaaatc gctataatga ccccgaagca gggttatgca 8880
   gcggaaaaga tccgtcgacc ctttccgacg ctcaccgggc tggttgccct cgccgctggg 8940
   ctggcggccg tctatggccc tgcaaacgcg ccagaaacgc cgtcgaagcc gtgtgcgaga 9000
   caccgcggcc ggccgccggc gttgtggata ccacgcggaa aacttggccc tcactgacag 9060
   atgaggggcg gacgttgaca cttgaggggc cgactcaccc ggcgcggcgt tgacagatga 9120
   ggggcaggct cgatttcggc cggcgacgtg gagctggcca gcctcgcaaa tcggcgaaaa 9180
   cgcctgattt tacgcgagtt tcccacagat gatgtggaca agcctgggga taagtgccct 9240
   gcggtattga cacttgaggg gcgcgactac tgacagatga ggggcgcgat ccttgacact 9300
   tgaggggcag agtgatgaca gatgaggggc gcacctattg acatttgagg ggctgtccac 9360
   aggcagaaaa tccagcattt gcaagggttt ccgcccgttt ttcggccacc gctaacctgt 9420
   cttttaacct gcttttaaac caatatttat aaaccttgtt tttaaccagg gctgcgccct 9480
   ggcgcgtgac cgcgcacgcc gaaggggggt gccccccctt ctcgaaccct cccggcccgc 9540
   taacgcgggc ctcccatccc cccaggggct gcgcccctcg gccgcgaacg gcctcacccc 9600
   aaaaatggca ggccaagctt gcttggtcgt tccgcgtgaa cgtcggctcg attgtacctg 9660
   cgttcaaata ctttgcgatc gtgttgcgcg cctgcccggt gcgtcggctg atctcacgga 9720
   tcgactgctt ctctcgcaac gccatccgac ggatgatgtt taaaagtccc atgtggatca 9780
   ctccgttgcc ccgtcgctca ccgtgttggg gggaaggtgc acatggctca gttctcaatg 9840
   gaaattatct gcctaaccgg ctcagttctg cgtagaaacc aacatgcaag ctccaccggg 9900
   tgcaaagcgg cagcggcggc aggatatatt caattgtaaa tggcttcatg tccgggaaat 9960
   ctacatggat cagcaatgag tatgatggtc aatatggaga aaaagaaaga gtaattacca 10020
   attttttttc aattcaaaaa tgtagatgtc cgcagcgtta ttataaaatg aaagtacatt 10080
   ttgataaaac gacaaattac gatccgtcgt atttataggc gaaagcaata aacaaattat 10140
   tctaattcgg aaatctttat ttcgacgtgt ctacattcac gtccaaatgg gggagatccg 10200
   tcgacctgca gtgaggtctg cctcgtgaag aaggtgttgc tgactcatac caggcctgaa 10260
   tcgccccatc atccagccag aaagtgaggg agccacggtt gatgagagct ttgttgtagg 10320
   tggaccagtt ggtgattttg aacttttgct ttgccacgga acggtctgcg ttgtcgggaa 10380
   gatgcgtgat ctgatccttc aactcagcaa aagttcgatt tattcaacaa agccacgttg 10440
   tgtctcaaaa tctctgatgt tacattgcac aagataaaaa tatatcatca tgaacaataa 10500
   aactgtctgc ttacataaac agtaatacaa ggggtgttat gagccatatt caacgggaaa 10560
   cgtcttgctc gaggccgcga ttaaattcca acatggatgc tgatttatat gggtataaat 10620
   gggctcgcga taatgtcggg caatcaggtg cgacaatcta tcgattgtat gggaagcccg 10680
   atgcgccaga gttgtttctg aaacatggca aaggtagcgt tgccaatgat gttacagatg 10740
   agatggtcag actaaactgg ctgacggaat ttatgcctct tccgaccatc aagcatttta 10800
   tccgtactcc tgatgatgca tggttactca ccactgcgat ccctgggaaa acagcattcc 10860
   aggtattaga agaatatcct gattcaggtg aaaatattgt tgatgcgctg gcagtgttcc 10920
   tgcgccggtt gcattcgatt cctgtttgta attgtccttt taacagcgat cgcgtatttc 10980
   gtctcgctca ggcgcaatca cgaatgaata acggtttggt tgatgcgagt gattttgatg 11040
   acgagcgtaa tggctggcct gttgaacaag tctggaaaga aatgcataaa cttttgccat 11100
   tctcaccgga ttcagtcgtc actcatggtg atttctcact tgataacctt atttttgacg 11160
   aggggaaatt aataggttgt attgatgttg gacgagtcgg aatcgcagac cgataccagg 11220
   atcttgccag cctatggaac tgcctcggtg agtgttctcc ttcattacag aaacggcttt 11280
   ttcaaaaata tggtattgat aatcctgata tgaataaatt gcagtttcat ttgatgctcg 11340
   atgagttttt ctaatcagaa ttggttaatt ggttgtaaca ctggcagagc attacgctga 11400
   cttgacggga cggcggcttt gttgaataaa tcgaactttt gctgagttga aggatcagat 11460
   cacgcatctt cccgacaacg cagaccgttc cgtggcaaag caaaagttca aaatcaccaa 11520
   ctggtccacc tacaacaaag ctctcatcaa ccgtggctcc ctcactttct ggctggatga 11580
   tggggcgatt caggcctggt atgagtcagc aacaccttct tcacgaggca gacctcagcg 11640
   cctgcaggtc gacggatctg ggggatctag cagatccgcg aggggatcga gcccgacata 11700
   tgccccggtt tcgttgcgac taacatgagt tcttggacaa atttgattgg acctgatgag 11760
   atgatccaac ccgaggatat agcaaagctc gttcgtgcag caatggaacg gccaaaccgt 11820
   gcttttgtcc ccaagaatga ggtgctatgc atgaaggaat ctacccgttg atgtccaaca 11880
   gtctcagggt taatgtctat gtatcttaaa taatgttgtc ggtattttgt aatctcatat 11940
   agattttcac tgtgcgacgc aaaaatatta aataaatatt attattatct acgtvttgat 12000
   tgagatatca tcaatattat aataaaaata tccattaaac acgatttgat acaaatgaca 12060
   gtcaataatc tgatttgaat atttattaat tgtaacgaat tacataaaga tcgaatagaa 12120
   aatactgcac tgcaaatgaa aattaacaca tactaataaa tgcgtcaaat atctttgcca 12180
   agatcaagcg gagtgagggc ctcatatccg gtctcagtta caagcacggt atccccgaag 12240
   cgcgctccac caatgccctc gacatagatg ccgggctcga cgctgaggac attgcctacc 12300
   ttgagcatgg tctcagcgcc ggctttaagc tcaatcccat cccaatctga atatcctatc 12360
   ccgcgcccag tccggtgtaa gaacgggtct gtccatccac ctctgttggg gtgggcgaag 12420
   aactccagca tgagatcccc gcgctggagg atcatccagc cggcgtcccg gaaaacgatt 12480
   ccgaagccca acctttcata gaaggcggcg gtggaatcga aatctcgtga tggcaggttg 12540
   ggcgtcgctt ggtcggtcat ttcgaacccc agagtcccgc tcagaagaac tcgtcaagaa 12600
   ggcgatagaa ggcgatgcgc tgcgaatcgg gagcggcgat accgtaaagc acgaggaagc 12660
   ggtcagccca ttcgccgcca agctcttcag caatatcacg ggtagccaac gctatgtcct 12720
   gatagcggtc cgccacaccc agccggccac agtcgatgaa tccagaaaag cggccatttt 12780
   ccaccatgat attcggcaag caggcatcgc catgggtcac gacgagatcc tcgccgtcgg 12840
   gcatgcgcgc cttgagcctg gcgaacagtt cggctggcgc gagcccctga tgctcttcgt 12900
   ccagatcatc ctgatcgaca agaccggctt ccatccgagt acgtgctcgc tcgatgcgat 12960
   gtttcgcttg gtggtcgaat gggcaggtag ccggatcaag cgtatgcagc cgccgcattg 13020
   catcagccat gatggatact ttctcggcag gagcaaggtg agatgacagg agatcctgcc 13080
   ccggcacttc gcccaatagc agccagtccc ttcccgcttc agtgacaacg tcgagcacag 13140
   ctgcgcaagg aacgcccgtc gtggccagcc acgatagccg cgctgcctcg tcctgcagtt 13200
   cattcagggc accggacagg tcggtcttga caaaaagaac cgggcgcccc tgcgctgaca 13260
   gccggaacac ggcggcatca gagcagccga ttgtctgttg tgcccagtca tagccgaata 13320
   gcctctccac ccaagcggcc ggagaacctg cgtgcaatcc atcttgttca atcatgctaa 13380
   aggatctcga tccccgggct gattttctca gtctccagag atgtgtttaa ataggcagta 13440
   gccttttgat atcagccaca agtgtgtggg aatcttatct tcggatttca attaggaatt 13500
   aaccttattg aattctcttg aaaggaagtc cgcaaagtgg ttgtcggttc ctttaatgtg 13560
   ttcaacatca aatgaatagt ggctaagcca tgcttgccat ctgatgtttc ttccaagttt 13620
   cgaatctcct ttgtaattga gattaacgaa actcttgaaa tgagtattat ctgtcctaat 13680
   cagaaaatga acaggagtta gataaatact aaatttcttt atagtattta ttaccgccaa 13740
   tgtctctttg tcattgctgt ggtaattctt ttctgcagct ttaaagctca gatcttgcag 13800
   gtccccagat tagccttttc aatttcagaa agaatgctaa cccacagatg gttagagagg 13860
   cttacgcagc aggtctcatc aagacgatct acccgagcaa taatctccag gagatcaaat 13920
   accttcccaa gaaggttaaa gatgcagtca aaagattcag gactaactgc atcaagaaca 13980
   cagagaaaga tatatttctc aagatcagaa gtactattcc agtatggacg attcaaggct 14040
   tgcttcacaa accaaggcaa gtaatagaga ttggagtctc taaaaaggta gttcccactg 14100
   aatcaaaggc catggagtca aagattcaaa tagaggacct aacagaactc gccgtaaaga 14160
   ctggcgaaca gttcatacag agtctcttac gactcaatga caagaagaaa atcttcgtca 14220
   acatggtgga gcacgacacg cttgtctact ccaaaaatat caaagataca gtctcagaag 14280
   accaaagggc aattgagact tttcaacaaa gggtaatatc cggaaacctc ctcggattcc 14340
   attgcccagc tatctgtcac tttattgtga agatagtgga aaaggaaggt ggctcctaca 14400
   aatgccatca ttgcgataaa ggaaaggcca tcgttgaaga tgcctctgcc gacagtggtc 14460
   ccaaagatgg acccccaccc acgaggagca tcgtggaaaa agaagacgtt ccaaccacgt 14520
   cttcaaagca agtggattga tgtgatatct ccactgacgt aagggatgac gcacaatccc 14580
   actatccttc gcaagaccct tcctctatat aaggaagttc atttcatttg gagagaacac 14640
   gggggactct agaggatccg gatccgtcga ccatgagcaa aggagaagaa cttttcactg 14700
   gagttgtccc aattcttgtt gaattagatg gtgatgttaa tgggcacaaa ttttctgtca 14760
   gtggagaggg tgaaggtgat gctacatacg gaaagcttac ccttaaattt atttgcacta 14820
   ctggaaaact acctgttcca tggccaacac ttgtcactac tttctcttat ggtgttcaat 14880
   gcttttcccg ttatccggat catatgaagc ggcacgactt cttcaagagc gccatgcctg 14940
   agggatacgt gcaggagagg accatctctt tcaaggacga cgggaactac aagacgcgtg 15000
   ctgaagtcaa gtttgaggga gacaccctcg tcaacaggat cgagcttaag ggaatcgatt 15060
   tcaaggagga cggaaacatc ctcggccaca agttggaata caactacaac tcccacaacg 15120
   tatacatcac ggcagacaaa caaaagaatg gaatcaaagc taacttcaaa attcgccaca 15180
   acattgaaga tggatccgtt caactagcag accattatca acaaaatact ccaattggcg 15240
   atggccctgt ccttttacca gacaaccatt acctgtcgac acaatctgcc ctttcgaaag 15300
   atcccaacga aaagcgtgac cacatggtcc ttcttgagtt tgtaactgct gctgggatta 15360
   cacatggcat ggatgagcta tacaaataaa gatcctcgaa tttccccgat cgttcaaaca 15420
   tttggcaata aagtttctta agattgaatc ctgttgccgg tcttgcgatg attatcatat 15480
   aatttctgtt gaattacgtt aagcatgtaa taattaacat gtaatgcatg acgttattta 15540
   tgagatgggt ttttatgatt agagtcccgc aattatacat ttaatacgcg atagaaaaca 15600
   aaatatagcg cgcaaactag gataaattat cgcgcgcggt gtcatctatg ttactagatc 15660
   gatcgggaat tagatc                                                 15676
<210> 31
   <211> 17111
   <212> DNA
   <213> Artificial Sequence
<220>
   <223> Description of Artificial Sequence:pArActAf-bin
<400> 31
   gatctgcaag cgatcgcggc gcgccattta aatgcccggg cgtttaaacg cggccgctct 60
   agaactagtg gatcctgcaa gagttttaca agtgaaatta agtttaaaca tttataaata 120
   tataccaagt caagtgtgct ttttaaaaat tttgctgact tcataaaata tattaggaag 180
   tcagcttggc atggagctat ataatcttaa aaaggaatca aaaatatcga aaaatatatt 240
   agaagggaga ggactaatta atatttacca gctccaggga aatatattct ttggagctgg 300
   cctatcgttt taaaattttt tttcatggac gtcatatata aatctaaacc ccttctaaat 360
   atattagaaa ttaaagatat cgagatcctg caagagtttt acaagtgaaa ttaagtttaa 420
   acatttataa atatatacca agtcaagtgt gctttttaaa aattttgctg acttcataaa 480
   atatattagg aagtcagctt ggcatggagc tatataatct taaaaaggaa tcaaaaatat 540
   cgaaaaatat attagaaggg agaggactaa ttaatattta ccagctccag ggaaatatat 600
   tctttggagc tggcctatcg ttttaaaatt ttttttcatg gacgtcatat ataaatctaa 660
   accccttcta aatatattag aaattaaaga tatcgagatc ccccgggctg cagaattcgg 720
   cttgttttga cgagttcgga tgtagtagta gccattattt aatgtacata ctaatcgtga 780
   atagtgaata tgatgaaaca ttgtatctta ttgtataaat atccataaac acatcatgaa 840
   agacactttc tttcacggtc tgaattaatt atgatacaat tctaatagaa aacgaattaa 900
   attacgttga attgtatgaa atctaattga acaagccaac cacgacgacg actaacgttg 960
   cctggattga ctcggtttaa gttaaccact aaaaaaacgg agctgtcatg taacacgcgg 1020
   atcgagcagg tcacagtcat gaagccatca aagcaaaaga actaatccaa ggggctgaga 1080
   tgattaatta gtttaaaaat tagttaacac gagggaaaag gctgcctgac agccaggtca 1140
   cgttatcttt acctgtggtc gaaatgattc gtgtctgtcg attttaatta tttttttgaa 1200
   aggccgaaaa taaagttgta agagataaac ccgcctatat aaattcatat attttcctct 1260
   ccgctttgaa ttgtctcgtt gtcctcctca ctttcatcag ccgttttgaa tctccggcga 1320
   cttgacagag aagaacaagg aagaagacta agagagaaag taagagataa tccaggagat 1380
   tcattctccg ttttgaatct tcctcaatct catcttcttc cgctctttct ttccaaggta 1440
   ataggaactt tctggatcta ctttatttgc tggatctcga tcttgttttc tcaatttcct 1500
   tgagatctgg aattcgttta atttgggatc tgtgaacctc cactaaatct tttggtttta 1560
   ctagaatcga tctaagttga ccgatcagtt agctcgatta tagctaccag aatttggctt 1620
   gaccttgatg gagagatcca tgttcatgtt acctgggaaa tgatttgtat atgtgaaatg 1680
   aaatctgaac tgttgaagtt agattgaatc tgaacactgt caatgttaga ttgaatctga 1740
   acactgttta aggttagatg aagtttgtgt atagattctt cgaaacttta ggatttgtag 1800
   tgtcgtacgt tgaacagaaa gctatttctg attcaatcag ggtttatttg actgtattga 1860
   actctttttg tgtgtttgca gctgtagacc atggtccgtc ctgtagaaac cccaacccgt 1920
   gaaatcaaaa aactcgacgg cctgtgggca ttcagtctgg atcgcgaaaa ctgtggaatt 1980
   gatcagcgtt ggtgggaaag cgcgttacaa gaaagccggg caattgctgt gccaggcagt 2040
   tttaacgatc agttcgccga tgcagatatt cgtaattatg cgggcaacgt ctggtatcag 2100
   cgcgaagtct ttataccgaa aggttcggca ggccagcgta tcgtgctgcg tttcgatgcg 2160
   gtcactcatt acggcaaagt gtgggtcaat aatcaggaag tgatggagca tcagggcggc 2220
   tatacgccat ttgaagccga tgtcacgccg tatgttattg ccgggaaaag tgtacgtatc 2280
   accgtttgtg tgaacaacga actgaactgg cagactatcc cgccgggaat ggtgattacc 2340
   gacgaaaacg gcaagaaaaa gcagtcttac ttccatgatt tctttaacta tgccggaatc 2400
   catcgcagcg taatgctcta caccacgccg aacacctggg tggacgatat caccgtggtg 2460
   acgcatgtcg cgcaagactg taaccacgcg tctgttgact ggcaggtggt ggccaatggt 2520
   gatgtcagcg ttgaactgcg tgatgcggat caacaggtgg ttgcaactgg acaaggcact 2580
   agcgggactt tgcaagtggt gaatccgcac ctctggcaac cgggtgaagg ttatctctat 2640
   gaactgtgcg tcacagccaa aagccagaca gagtgtgata tctacccgct tcgcgtcggc 2700
   atccggtcag tggcagtgaa gggcgaacag ttcctgatta accacaaacc gttctacttt 2760
   actggctttg gtcgtcatga agatgcggac ttacgtggca aaggattcga taacgtgctg 2820
   atggtgcacg accacgcatt aatggactgg attggggcca actcctaccg tacctcgcat 2880
   tacccttacg ctgaagagat gctcgactgg gcagatgaac atggcatcgt ggtgattgat 2940
   gaaactgctg ctgtcggctt taacctctct ttaggcattg gtttcgaagc gggcaacaag 3000
   ccgaaagaac tgtacagcga agaggcagtc aacggggaaa ctcagcaagc gcacttacag 3060
   gcgattaaag agctgatagc gcgtgacaaa aaccacccaa gcgtggtgat gtggagtatt 3120
   gccaacgaac cggatacccg tccgcaagtg cacgggaata tttcgccact ggcggaagca 3180
   acgcgtaaac tcgacccgac gcgtccgatc acctgcgtca atgtaatgtt ctgcgacgct 3240
   cacaccgata ccatcagcga tctctttgat gtgctgtgcc tgaaccgtta ttacggatgg 3300
   tatgtccaaa gcggcgattt ggaaacggca gagaaggtac tggaaaaaga acttctggcc 3360
   tggcaggaga aactgcatca gccgattatc atcaccgaat acggcgtgga tacgttagcc 3420
   gggctgcact caatgtacac cgacatgtgg agtgaagagt atcagtgtgc atggctggat 3480
   atgtatcacc gcgtctttga tcgcgtcagc gccgtcgtcg gtgaacaggt atggaatttc 3540
   gccgattttg cgacctcgca aggcatattg cgcgttggcg gtaacaagaa agggatcttc 3600
   actcgcgacc gcaaaccgaa gtcggcggct tttctgctgc aaaaacgctg gactggcatg 3660
   aacttcggtg aaaaaccgca gcagggaggc aaacaatgaa tcaacaactc tcctggcgca 3720
   ccatcgtcgg ctacagcctc ggtggggaat tggagagctc gaatttcccc gatcgttcaa 3780
   acatttggca ataaagtttc ttaagattga atcctgttgc cggtcttgcg atgattatca 3840
   tataatttct gttgaattac gttaagcatg taataattaa catgtaatgc atgacgttat 3900
   ttatgagatg ggtttttatg attagagtcc cgcaattata catttaatac gcgatagaaa 3960
   acaaaatata gcgcgcaaac taggataaat tatcgcgcgc ggtgtcatct atgttactag 4020
   atcgatcggg aattgtttgg gggatctcga tatctttaat ttctaatata tttagaaggg 4080
   gtttagattt atatatgacg tccatgaaaa aaaattttaa aacgataggc cagctccaaa 4140
   gaatatattt ccctggagct ggtaaatatt aattagtcct ctcccttcta atatattttt 4200
   cgatattttt gattcctttt taagattata tagctccatg ccaagctgac ttcctaatat 4260
   attttatgaa gtcagcaaaa tttttaaaaa gcacacttga cttggtatat atttataaat 4320
   gtttaaactt aatttcactt gtaaaactct tgcaggatct cgatatcttt aatttctaat 4380
   atatttagaa ggggtttaga tttatatatg acgtccatga aaaaaatttt taaaacgata 4440
   ggccagctcc aaagaatata tttccctgga gctggtaaat attaattagt cctctccctt 4500
   ctaatatatt tttcgatatt tttgattcct ttttaagatt atatagctcc atgccaagct 4560
   gacttcctaa tatattttat gaagtcagca aaatttttaa aaagcacact tgacttggta 4620
   tatatttata aatgtttaaa cttaatttca cttgtaaaac tcttgcagga tccactagtt 4680
   ctagagcggc cgcttaatta aggccggcct gcaggcatgc aagctcggat ctcacctacg 4740
   atggggggca tcgcaccggt gagtaatatt gtacggctaa gagcgaattt ggcctgtaga 4800
   cctcaattgc gagctttcta atttcaaact attcgggcct aacttttggt gtgatgatgc 4860
   tgactggcag gatatatacc gttgtaattt gagctcgtgt gaataagtcg ctgtgtatgt 4920
   ttgtttgatt gtttctgttg gagtgcagcc catttcaccg gacaagtcgg ctagattgat 4980
   ttagccctga tgaactgccg aggggaagcc atcttgagcg cggaatggga atggatttcg 5040
   ttgtacaacg agacgacaga acacccacgg gaccgagctt cgcaagcttg ttgtaactga 5100
   aaaaggaaaa ttattgtgcc aggcagttga aagtcagcac cttttaacga gtgctgaaat 5160
   gacggctaaa tgggaaacgt atttaaaaaa aatcggtaaa agagaaggca atcaagagaa 5220
   ctttattacg aatatcaaaa aattcattgt tcatttactg gaagctgtac ctaacgatat 5280
   agaaaaacta aatttttctg attaccagga acagaaagaa aaagaagcag aaaaaagtat 5340
   tgtaggaaaa tgtcctaagt gtggcaacaa tattgtatta aaaaaatcgt tttatggttg 5400
   ttcaaattat cctgaatgta agtttacttt agctgaacat tttagaaaga aaaaactcac 5460
   caaaacaaat gtaaaagaat tactagaggg aaaagaaacc ctggtaaaag gaatcaaaac 5520
   gaaagataga aagtcctaca atgccgttgt aaaaatcgga gaaaagggat atattgattt 5580
   tatatctttc tcaaaataaa cataaaagcc ctttaaagag ggcttttata tattaatcac 5640
   aaatcactta tcacaaatca caagtgattt gtgattgttg atgataaaat aagaataaga 5700
   agaaatagaa agaagtgagt gattgtggga aatttaggcg cacaaaaaga aaaacgaaat 5760
   gatacaccaa tcagtgcaaa aaaagatata atgggagata agacggttcg tgttcgtgct 5820
   gacttgcacc atatcataaa aatcgaaaca gcaaagaatg gcggaaacgt aaaagaagtt 5880
   atggaaataa gacttagaag caaacttaag agtgtgttga tagtgcagta tcttaaaatt 5940
   ttgtataata ggaattgaag ttaaattaga tgctaaaaat ttgtaattaa gaaggagtga 6000
   ttacatgaac aaaaatataa aatattctca aaacttttta acgagtgaaa aagtactcaa 6060
   ccaaataata aaacaattga atttaaaaga aaccgatacc gtttacgaaa ttggaacagg 6120
   taaagggcat ttaacgacga aactggctaa aataagtaaa caggtaacgt ctattgaatt 6180
   agacagtcat ctattcaact tatcgtcaga aaaattaaaa ctgaatactc gtgtcacttt 6240
   aattcaccaa gatattctac agtttcaatt ccctaacaaa cagaggtata aaattgttgg 6300
   gagtattcct taccatttaa gcacacaaat tattaaaaaa gtggtttttg aaagccatgc 6360
   gtctgacatc tatctgattg ttgaagaagg attctacaag cgtaccttgg atattcaccg 6420
   aacactaggg ttgctcttgc acactcaagt ctcgattcag caattgctta agctgccagc 6480
   ggaatgcttt catcctaaac caaaagtaaa cagtgtctta ataaaactta cccgccatac 6540
   cacagatgtt ccagataaat attggaagct atatacgtac tttgtttcaa aatgggtcaa 6600
   tcgagaatat cgtcaactgt ttactaaaaa tcagtttcat caagcaatga aacacgccaa 6660
   agtaaacaat ttaagtaccg ttacttatga gcaagtattg tctattttta atagttatct 6720
   attatttaac gggaggaaat aattctatga gtcgcttttg taaatttgga aagttacacg 6780
   ttactaaagg gaatgtagat aaattattag gtatactact gacagcttcc aaggagctaa 6840
   agaggtccct agcgcctacg gggaatttgt atcgataagg ggtacaaatt cccactaagc 6900
   gctcgggggc tgagaaagcc cagtaaggaa acaactgtag gttcgagtcg cgagatcccc 6960
   cggaaccaaa ggaagtaggt taaacccgct ccgatcaggc cgagccacgc caggccgaga 7020
   acattggttc ctgtaggcat cgggattggc ggatcaaaca ctaaagctac tggaacgagc 7080
   agaagtcctc cggccgccag ttgccaggcc gtaaaggtga gcagaggcac gggaggttgc 7140
   cacttgcggg tcagcacggt tccgaacgcc atggaaaccg cccccgccag gcccgctgcg 7200
   acgccgacag gatctagcgc tgcgtttggt gtcaacacca acagcgccac gcccgcagtt 7260
   ccgcaaatag cccccaggac cgccatcaat cgtatcgggc tacctagcag agcggcagag 7320
   atgaacacga ccatcagcgg ctgcacagcg cctaccgtcg ccgcgacccg cccggcaggc 7380
   ggtagaccga aataaacaac aagctccaga atagcgaaat attaagtgcg ccgaggatga 7440
   agatgcgcat ccaccagatt cccgttggaa tctgtcggac gatcatcacg agcaataaac 7500
   ccgccggcaa cgcccgcagc agcataccgg cgacccctcg gcctcgctgt tcgggctcca 7560
   cgaaaacgcc ggacagatgc gccttgtgag cgtccttggg gccgtcctcc tgtttgaaga 7620
   ccgacagccc aatgatctcg ccgtcgatgt aggcgccgaa tgccacggca tctcgcaacc 7680
   gttcagcgaa cgcctccatg ggctttttct cctcgtgctc gtaaacggac ccgaacatct 7740
   ctggagcttt cttcagggcc gacaatcgga tctcgcggaa atcctgcacg tcggccgctc 7800
   caagccgtcg aatctgagcc ttaatcacaa ttgtcaattt taatcctctg tttatcggca 7860
   gttcgtagag cgcgccgtgc gcccgagcga tactgagcga agcaagtgcg tcgagcagtg 7920
   cccgcttgtt cctgaaatgc cagtaaagcg ctggctgctg aacccccagc cggaactgac 7980
   cccacaaggc cctagcgttt gcaatgcacc aggtcatcat tgacccaggc gtgttccacc 8040
   aggccgctgc ctcgcaactc ttcgcaggct tcgccgacct gctcgcgcca cttcttcacg 8100
   cgggtggaat ccgatccgca catgaggcgg aaggtttcca gcttgagcgg gtacggctcc 8160
   cggtgcgagc tgaaatagtc gaacatccgt cgggccgtcg gcgacagctt gcggtacttc 8220
   tcccatatga atttcgtgta gtggtcgcca gcaaacagca cgacgatttc ctcgtcgatc 8280
   aggacctggc aacgggacgt ttcttgccca cggtccagga cgcggaagcg gtgcagcagc 8340
   gacaccgatt ccaggtgccc aacgcggtcg gacgtgaagc ccatcgccgt cgcctgtagg 8400
   cgcgacaggc attcctcggc cttcgtgtaa taccggccat tgatcgacca gcccaggtcc 8460
   tggcaaagct cgtagaacgt gaaggtgatc ggctcgccga taggggtgcg cttcgcgtac 8520
   tccaacacct gctgccacac cagttcgtca tcgtcggccc gcagctcgac gccggtgtag 8580
   gtgatcttca cgtccttgtt gacgtggaaa atgaccttgt tttgcagcgc ctcgcgcggg 8640
   attttcttgt tgcgcgtggt gaacagggca gagcgggccg tgtcgtttgg catcgctcgc 8700
   atcgtgtccg gccacggcgc aatatcgaac aaggaaagct gcatttcctt gatctgctgc 8760
   ttcgtgtgtt tcagcaacgc ggcctgcttg gcctcgctga cctgttttgc caggtcctcg 8820
   ccggcggttt ttcgcttctt ggtcgtcata gttcctcgcg tgtcgatggt catcgacttc 8880
   gccaaacctg ccgcctcctg ttcgagacga cgcgaacgct ccacggcggc cgatggcgcg 8940
   ggcagggcag ggggagccag ttgcacgctg tcgcgctcga tcttggccgt agcttgctgg 9000
   accatcgagc cgacggactg gaaggtttcg cggggcgcac gcatgacggt gcggcttgcg 9060
   atggtttcgg catcctcggc ggaaaacccc gcgtcgatca gttcttgcct gtatgccttc 9120
   cggtcaaacg tccgattcat tcaccctcct tgcgggattg ccccgactca cgccggggca 9180
   atgtgccctt attcctgatt tgacccgcct ggtgccttgg tgtccagata atccacctta 9240
   tcggcaatga agtcggtccc gtagaccgtc tggccgtcct tctcgtactt ggtattccga 9300
   atcttgccct gcacgaatac cagcgacccc ttgcccaaat acttgccgtg ggcctcggcc 9360
   tgagagccaa aacacttgat gcggaagaag tcggtgcgct cctgcttgtc gccggcatcg 9420
   ttgcgccact cttcattaac cgctatatcg aaaattgctt gcggcttgtt agaattgcca 9480
   tgacgtacct cggtgtcacg ggtaagatta ccgataaact ggaactgatt atggcnnctc 9540
   gaaattccct cggtcttgcc ttgctcgtcg gtgatgtact tcaccagctc cgcgaagtcg 9600
   ctcttcttga tggagcgcat ggggacgtgc ttggcaatca cgcgcacccc ccggccgttt 9660
   tagcggctaa aaaagtcatg gctctgccct cgggcggacc acgcccatca tgaccttgcc 9720
   aagctcgtcc tgcttctctt cgatcttcgc cagcagggcg aggatcgtgg catcaccgaa 9780
   ccgcgccgtg cgcgggtcgt cggtgagcca gagtttcagc aggccgccca ggcggcccag 9840
   gtcgccattg atgcgggcca gctcgcggac gtgctcatag tccacgacgc ccgtgatttt 9900
   gtagccctgg ccgacggcca gcaggtaggc cgacaggctc atgccggccg ccgccgcctt 9960
   ttcctcaatc gctcttcgtt cgtctggaag gcagtacacc ttgataggtg ggctgccctt 10020
   cctggttggc ttggtttcat cagccatccg cttgccctca tctgttacgc cggcggtagc 10080
   cggccagcct cgcagagcag gattcccgtt gagcaccgcc aggtgcgaat aagggacagt 10140
   gaagaaggaa cacccgctcg cgggtgggcc tacttcacct atcctgcccg gctgacgccg 10200
   ttggatacac caaggaaagt ctacacgaac cctttggcaa aatcctgtat atcgtgcgaa 10260
   aaaggatgga tataccgaaa aaatcgctat aatgaccccg aagcagggtt atgcagcgga 10320
   aaagatccgt cgaccctttc cgacgctcac cgggctggtt gccctcgccg ctgggctggc 10380
   ggccgtctat ggccctgcaa acgcgccaga aacgccgtcg aagccgtgtg cgagacaccg 10440
   cggccggccg ccggcgttgt ggataccacg cggaaaactt ggccctcact gacagatgag 10500
   gggcggacgt tgacacttga ggggccgact cacccggcgc ggcgttgaca gatgaggggc 10560
   aggctcgatt tcggccggcg acgtggagct ggccagcctc gcaaatcggc gaaaacgcct 10620
   gattttacgc gagtttccca cagatgatgt ggacaagcct ggggataagt gccctgcggt 10680
   attgacactt gaggggcgcg actactgaca gatgaggggc gcgatccttg acacttgagg 10740
   ggcagagtga tgacagatga ggggcgcacc tattgacatt tgaggggctg tccacaggca 10800
   gaaaatccag catttgcaag ggtttccgcc cgtttttcgg ccaccgctaa cctgtctttt 10860
   aacctgcttt taaaccaata tttataaacc ttgtttttaa ccagggctgc gccctggcgc 10920
   gtgaccgcgc acgccgaagg ggggtgcccc cccttctcga accctcccgg cccgctaacg 10980
   cgggcctccc atccccccag gggctgcgcc cctcggccgc gaacggcctc accccaaaaa 11040
   tggcaggcca agcttgcttg gtcgttccgc gtgaacgtcg gctcgattgt acctgcgttc 11100
   aaatactttg cgatcgtgtt gcgcgcctgc ccggtgcgtc ggctgatctc acggatcgac 11160
   tgcttctctc gcaacgccat ccgacggatg atgtttaaaa gtcccatgtg gatcactccg 11220
   ttgccccgtc gctcaccgtg ttgcggggaa ggtgcacatg gctcagttct caatggaaat 11280
   tatctgccta accggctcag ttctgcgtag aaaccaacat gcaagctcca ccgggtgcaa 11340
   agcggcagcg gcggcaggat atattcaatt gtaaatggct tcatgtccgg gaaatctaca 11400
   tggatcagca atgagtatga tggtcaatat ggagaaaaag aaagagtaat taccaatttt 11460
   ttttcaattc aaaaatgtag atgtccgcag cgttattata aaatgaaagt acattttgat 11520
   aaaacgacaa attacgatcc gtcgtattta taggcgaaag caataaacaa attattctaa 11580
   ttcggaaatc tttatttcga cgtgtctaca ttcacgtcca aatgggggag atccgtcgac 11640
   ctgcagtgag gtctgcctcg tgaagaaggt gttgctgact cataccaggc ctgaatcgcc 11700
   ccatcatcca gccagaaagt gagggagcca cggttgatga gagctttgtt gtaggtggac 11760
   cagttggtga ttttgaactt ttgctttgcc acggaacggt ctgcgttgtc gggaagatgc 11820
   gtgatctgat ccttcaactc agcaaaagtt cgatttattc aacaaagcca cgttgtgtct 11880
   caaaatctct gatgttacat tgcacaagat aaaaatatat catcatgaac aataaaactg 11940
   tctgcttaca taaacagtaa tacaaggggt gttatgagcc atattcaacg ggaaacgtct 12000
   tgctcgaggc cgcgattaaa ttccaacatg gatgctgatt tatatgggta taaatgggct 12060
   cgcgataatg tcgggcaatc aggtgcgaca atctatcgat tgtatgggaa gcccgatgcg 12120
   ccagagttgt ttctgaaaca tggcaaaggt agcgttgcca atgatgttac agatgagatg 12180
   gtcagactaa actggctgac ggaatttatg cctcttccga ccatcaagca ttttatccgt 12240
   actcctgatg atgcatggtt actcaccact gcgatccctg ggaaaacagc attccaggta 12300
   ttagaagaat atcctgattc aggtgaaaat attgttgatg cgctggcagt gttcctgcgc 12360
   cggttgcatt cgattcctgt ttgtaattgt ccttttaaca gcgatcgcgt atttcgtctc 12420
   gctcaggcgc aatcacgaat gaataacggt ttggttgatg cgagtgattt tgatgacgag 12480
   cgtaatggct ggcctgttga acaagtctgg aaagaaatgc ataaactttt gccattctca 12540
   ccggattcag tcgtcactca tggtgatttc tcacttgata accttatttt tgacgagggg 12600
   aaattaatag gttgtattga tgttggacga gtcggaatcg cagaccgata ccaggatctt 12660
   gccagcctat ggaactgcct cggtgagtgt tctccttcat tacagaaacg gctttttcaa 12720
   aaatatggta ttgataatcc tgatatgaat aaattgcagt ttcatttgat gctcgatgag 12780
   tttttctaat cagaattggt taattggttg taacactggc agagcattac gctgacttga 12840
   cgggacggcg gctttgttga ataaatcgaa cttttgctga gttgaaggat cagatcacgc 12900
   atcttcccga caacgcagac cgttccgtgg caaagcaaaa gttcaaaatc accaactggt 12960
   ccacctacaa caaagctctc atcaaccgtg gctccctcac tttctggctg gatgatgggg 13020
   cgattcaggc ctggtatgag tcagcaacac cttcttcacg aggcagacct cagcgcctgc 13080
   aggtcgacgg atctggggga tctagcagat ccgcgagggg atcgagcccg acatatgccc 13140
   cggtttcgtt gcgactaaca tgagttcttg gacaaatttg attggacctg atgagatgat 13200
   ccaacccgag gatatagcaa agctcgttcg tgcagcaatg gaacggccaa accgtgcttt 13260
   tgtccccaag aatgaggtgc tatgcatgaa ggaatctacc cgttgatgtc caacagtctc 13320
   agggttaatg tctatgtatc ttaaataatg ttgtcggtat tttgtaatct catatagatt 13380
   ttcactgtgc gacgcaaaaa tattaaataa atattattat tatctacgtv ttgattgaga 13440
   tatcatcaat attataataa aaatatccat taaacacgat ttgatacaaa tgacagtcaa 13500
   taatctgatt tgaatattta ttaattgtaa cgaattacat aaagatcgaa tagaaaatac 13560
   tgcactgcaa atgaaaatta acacatacta ataaatgcgt caaatatctt tgccaagatc 13620
   aagcggagtg agggcctcat atccggtctc agttacaagc acggtatccc cgaagcgcgc 13680
   tccaccaatg ccctcgacat agatgccggg ctcgacgctg aggacattgc ctaccttgag 13740
   catggtctca gcgccggctt taagctcaat cccatcccaa tctgaatatc ctatcccgcg 13800
   cccagtccgg tgtaagaacg ggtctgtcca tccacctctg ttggggtggg cgaagaactc 13860
   cagcatgaga tccccgcgct ggaggatcat ccagccggcg tcccggaaaa cgattccgaa 13920
   gcccaacctt tcatagaagg cggcggtgga atcgaaatct cgtgatggca ggttgggcgt 13980
   cgcttggtcg gtcatttcga accccagagt cccgctcaga agaactcgtc aagaaggcga 14040
   tagaaggcga tgcgctgcga atcgggagcg gcgataccgt aaagcacgag gaagcggtca 14100
   gcccattcgc cgccaagctc ttcagcaata tcacgggtag ccaacgctat gtcctgatag 14160
   cggtccgcca cacccagccg gccacagtcg atgaatccag aaaagcggcc attttccacc 14220
   atgatattcg gcaagcaggc atcgccatgg gtcacgacga gatcctcgcc gtcgggcatg 14280
   cgcgccttga gcctggcgaa cagttcggct ggcgcgagcc cctgatgctc ttcgtccaga 14340
   tcatcctgat cgacaagacc ggcttccatc cgagtacgtg ctcgctcgat gcgatgtttc 14400
   gcttggtggt cgaatgggca ggtagccgga tcaagcgtat gcagccgccg cattgcatca 14460
   gccatgatgg atactttctc ggcaggagca aggtgagatg acaggagatc ctgccccggc 14520
   acttcgccca atagcagcca gtcccttccc gcttcagtga caacgtcgag cacagctgcg 14580
   caaggaacgc ccgtcgtggc cagccacgat agccgcgctg cctcgtcctg cagttcattc 14640
   agggcaccgg acaggtcggt cttgacaaaa agaaccgggc gcccctgcgc tgacagccgg 14700
   aacacggcgg catcagagca gccgattgtc tgttgtgccc agtcatagcc gaatagcctc 14760
   tccacccaag cggccggaga acctgcgtgc aatccatctt gttcaatcat gctaaaggat 14820
   ctcgatcccc gggctgattt tctcagtctc cagagatgtg tttaaatagg cagtagcctt 14880
   ttgatatcag ccacaagtgt gtgggaatct tatcttcgga tttcaattag gaattaacct 14940
   tattgaattc tcttgaaagg aagtccgcaa agtggttgtc ggttccttta atgtgttcaa 15000
   catcaaatga atagtggcta agccatgctt gccatctgat gtttcttcca agtttcgaat 15060
   ctcctttgta attgagatta acgaaactct tgaaatgagt attatctgtc ctaatcagaa 15120
   aatgaacagg agttagataa atactaaatt tctttatagt atttattacc gccaatgtct 15180
   ctttgtcatt gctgtggtaa ttcttttctg cagctttaaa gctcagatct tgcaggtccc 15240
   cagattagcc ttttcaattt cagaaagaat gctaacccac agatggttag agaggcttac 15300
   gcagcaggtc tcatcaagac gatctacccg agcaataatc tccaggagat caaatacctt 15360
   cccaagaagg ttaaagatgc agtcaaaaga ttcaggacta actgcatcaa gaacacagag 15420
   aaagatatat ttctcaagat cagaagtact attccagtat ggacgattca aggcttgctt 15480
   cacaaaccaa ggcaagtaat agagattgga gtctctaaaa aggtagttcc cactgaatca 15540
   aaggccatgg agtcaaagat tcaaatagag gacctaacag aactcgccgt aaagactggc 15600
   gaacagttca tacagagtct cttacgactc aatgacaaga agaaaatctt cgtcaacatg 15660
   gtggagcacg acacgcttgt ctactccaaa aatatcaaag atacagtctc agaagaccaa 15720
   agggcaattg agacttttca acaaagggta atatccggaa acctcctcgg attccattgc 15780
   ccagctatct gtcactttat tgtgaagata gtggaaaagg aaggtggctc ctacaaatgc 15840
   catcattgcg ataaaggaaa ggccatcgtt gaagatgcct ctgccgacag tggtcccaaa 15900
   gatggacccc cacccacgag gagcatcgtg gaaaaagaag acgttccaac cacgtcttca 15960
   aagcaagtgg attgatgtga tatctccact gacgtaaggg atgacgcaca atcccactat 16020
   ccttcgcaag acccttcctc tatataagga agttcatttc atttggagag aacacggggg 16080
   actctagagg atccggatcc gtcgaccatg agcaaaggag aagaactttt cactggagtt 16140
   gtcccaattc ttgttgaatt agatggtgat gttaatgggc acaaattttc tgtcagtgga 16200
   gagggtgaag gtgatgctac atacggaaag cttaccctta aatttatttg cactactgga 16260
   aaactacctg ttccatggcc aacacttgtc actactttct cttatggtgt tcaatgcttt 16320
   tcccgttatc cggatcatat gaagcggcac gacttcttca agagcgccat gcctgaggga 16380
   tacgtgcagg agaggaccat ctctttcaag gacgacggga actacaagac gcgtgctgaa 16440
   gtcaagtttg agggagacac cctcgtcaac aggatcgagc ttaagggaat cgatttcaag 16500
   gaggacggaa acatcctcgg ccacaagttg gaatacaact acaactccca caacgtatac 16560
   atcacggcag acaaacaaaa gaatggaatc aaagctaact tcaaaattcg ccacaacatt 16620
   gaagatggat ccgttcaact agcagaccat tatcaacaaa atactccaat tggcgatggc 16680
   cctgtccttt taccagacaa ccattacctg tcgacacaat ctgccctttc gaaagatccc 16740
   aacgaaaagc gtgaccacat ggtccttctt gagtttgtaa ctgctgctgg gattacacat 16800
   ggcatggatg agctatacaa ataaagatcc tcgaatttcc ccgatcgttc aaacatttgg 16860
   caataaagtt tcttaagatt gaatcctgtt gccggtcttg cgatgattat catataattt 16920
   ctgttgaatt acgttaagca tgtaataatt aacatgtaat gcatgacgtt atttatgaga 16980
   tgggttttta tgattagagt cccgcaatta tacatttaat acgcgataga aaacaaaata 17040
   tagcgcgcaa actaggataa attatcgcgc gcggtgtcat ctatgttact agatcgatcg 17100
   ggaattagat c 17111
<210> 32
   <211> 17116
   <212> DNA
   <213> Artificial Sequence
<220>
   <223> Description of Artificial Sequence:pAfActAf-bin
<400> 32
<210> 33
   <211> 646
   <212> DNA
   <213> Artificial Sequence
<220>
   <223> Description of Artificial Sequence: 3' MAR in pAf-Act2-Af
<400> 33
   gggggatctc gatatcttta atttctaata tatttagaag gggtttagat ttatatatga 60
   cgtccatgaa aaaaaatttt aaaacgatag gccagctcca aagaatatat ttccctggag 120
   ctggtaaata ttaattagtc ctctccctcc taatatattt ttcgatattt ttgattcctt 180
   tttaagatta tatagctcca tgccaagctg acttcctaat atattttatg aagtcagcaa 240
   aatttttaaa aagcacactt gacttggtat atatttataa atgtttaaac ttaatttcac 300
   ttgtaaaact cttgcaggat ctcgatatct ttaatttcta atatatttag aaggggttta 360
   gatttatata tgacgtccat gaaaaaaaat tttaaaacga taggccagct ccaaagaata 420
   tatttccctg gagctggtaa atattaatta gtcctctccc ttctaatata tttttcgata 480
   tttttgattc ctttttaaga ttatatagct ccatgccaag ctgacttcct aatatatttt 540
   atgaagtcag caaaattttt aaaaagcaca cttgacttgg tatatattta taaatgttta 600
   aacttaattt cacttgtaaa actcttgcag gatccactag ttctag                646

## Description

The present invention relates to plant molecular biology, and in particular to technology for enhancing the expression of genes introduced in transformed plant cells.

Through the use of recombinant DNA technology and genetic engineering, it has become possible to introduce desired DNA sequences into plant cells to allow for the expression of proteins of interest. Plants with genetically engineered traits, such as, for example, insect resistance, disease resistance, drought resistance, herbicide resistance, or metabolic alterations that increase or modify production of useful plant products, offer great promise of improving agriculture.

Obtaining desired levels of expression of DNA introduced into plant cells remains a challenge. One problem, referred to as "position effect" variation, is the variation in expression of the same gene in independent transformants. The use of naturally occurring DNA sequences called matrix attachment regions or scaffold attachment regions to combat this problem was proposed in U.S. Patent 5,773,689 and in WO 94/24293.

The present invention provides a novel synthetic DNA molecule comprising bp 11 to 309 of SEQ ID NO: 1 that is useful as a matrix attachment region to increase expression of genes introduced in transformed plants.

In another of its aspects, the invention provides a DNA construct comprising, in the 5' to 3' direction: a transcription initiation region functional in plant cells, a structural gene operatively associated with the transcription initiation region, a 3' untranslated region, and a matrix attachment region comprised of bp 11 to 309 of SEQ ID NO: 1 positioned either 5' to said transcription initiation region or 3' to said structural gene. In a preferred embodiment, a first matrix attachment region comprised of bp 11 to 309 of SEQ ID NO: 1 is positioned 5' to said transcription initiation region and a second matrix attachment region comprised of bp 11 to 309 of SEQ ID NO: 1 is positioned 3' to said 3' untranslated region.

In a particularly preferred embodiment, the matrix attachment region of the invention comprises two or more tandem copies of bp 11 to 309 of SEQ ID NO:1.

### BRIEF DESCRIPTION OF THE DRAWINGS

FIG. 1 is a diagram showing the strategy for assembling the artificial MAR of SEQ ID NO:1.
FIG. 2 is a schematic representation of the rice transformation construct ArGOS2Af, which contains the MAR dimer.
FIG. 3 is a graph comparing relative GUS activity for multiple rice transformation events using non-MAR containing construct GOS2 and construct ArGOS2Af, which contains an artificial MAR dimer.
FIG. 4 is a graph showing the effect of the artificial MAR on ranges of expression of the GUS reporter gene in transgenic rice plants.
FIG. 5 is a schematic representation of the *Arabidopsis* transformation constructs ArAct2Af and aaaaAfAct2Af. ArAct2Af contains copies of the MAR dimer in opposite orientations flanking the reporter gene. AfAct2Af contains copies of the MAR dimer in the same orientation flanking the reporter.gene.
FIG. 6 is a graph comparing relative GUS activity for multiple *Arabidopsis* transformation events using non-MAR containing construct Act2 and constructs ArAct2Af and AfAct2Af, which contain an artificial MAR dimer.
FIG 7 is a graph showing the effect of the artificial MAR on the range of expression of the GUS reporter gene in transgenic *Arabidopsis* plants.

### DESCRIPTION OF THE SEQUENCES

SEQ ID NO:1 describes the artificial MAR of the invention.
SEQ ID NOS:2 to 4 describe ARBP sites.
SEQ ID NO:5 describes an ATF site.
SEQ ID NO:6 describes a BEAF-32 site.
SEQ ID NOS:7 to 9 describe topoisomerase II sites.
SEQ ID NO:10 describes an unwinding sequence.
SEQ ID NOS:11 to 17 describe SATB I sites.
SEQ ID NO:18 describes exemplary bending DNA.
SEQ ID NO:19 describes an exemplary A/T tract.
SEQ ID NO:20 describes synthetic MAR-A.
SEQ ID NO:21 describes synthetic MAR-B.
SEQ ID NO:22 describes synthetic MAR-C.
SEQ ID NO:23 describes synthetic MAR-D.
SEQ ID NO:24 describes synthetic MAR-E.
SEQ ID NO:25 describes synthetic MAR-F.
SEQ ID NO:26 describes the 3' MAR dimer in pArGOS2Af-hpt and ArAct2Af-bin
SEQ ID NO:27 describes rice transformation vector pGOS2-hpt.
SEQ ID NO:28 describes rice transformation vector pArGOS2Af-hpt.
SEQ ID NO:29 describes the 5' MAR dimer in pArGOS2Af-hpt and ArAct2Af-bin.
SEQ ID NO:30 describes dicot transformation vector pAct2-bin.
SEQ ID NO:31 describes dicot transformation vector pArAct2Af-bin.
SEQ ID NO:32 describes dicot transformation vector pAfAct2Af-bin.
SEQ ID NO:33 describes the 3' MAR dimer in pAfAct2Af-bin.

### DETAILED DESCRIPTION OF THE INVENTION

Eukaryotic nuclei are-highly organized structures in which the entire genetic information has to be accessible in an orderly manner for replication, transcription and other cellular events (Lewin, 1994; Dillon and Grosveld, 1994; Jackson, 1995; Wolffe, 1994). Genes are typically organized in chromatin loops of various sizes that are attached to the proteinaceous nuclear matrix at locations known as matrix attachment regions (MARs). MARs are often located in non-transcribed regions of genes and are thought to form the physical boundaries of individual DNA loops. In several cases, MARs were shown to reduce position effect in transgenic organisms. The chicken lysozyme MAR was shown to increase expression, reduce variance and make expression of an adjacent gene copy number dependent in stably transfected cells (Stief *et al*., 1989) in transgenic mice (Bonifer *et al*., 1990, McKnight *et al*., 1992) and in transgenic tobacco plants (Mlynárová *et al*., 1994; Mlynárová *et al*., 1995).

However, not all MARs have these effects on gene expression. Two minimal *Drosophila* MARs (one located between the histone H1 and H3 genes, and the other near the heat shock HSP70 genes) stimulated expression more than 10-fold in stably transformed cells, but the presence of these MARs did not reduce position effect (Poljak et al., 1994). MARs from the apolipoprotein domain increased expression and reduced position effect in low-copy transformants, but expression in multicopy transformants was strongly repressed. (Kalos and Fournier, 1995). When a *Drosophila ftz* MAR was placed in a different chromosomal location, it did not reorganize chromatin structure and the chromatin fragment containing the MAR could be easily eluted from the nucleus, indicating that introduced MARs do not necessarily form chromatin domains (Eggert and Jack, 1991). In contrast, MARs flanking the immunoglobulin m heavy chain locus enhancer were required for high levels of expression and the formation of an extended DNase I sensitive domain in transgenic B lymphocytes, but not in stably transfected tissue culture cells (Forrester *et al*., 1994).

Results using MARs in transgenic plants have been similarly complex (Spiker and Thompson, 1996). A yeast MAR increased expression levels in stably transformed tobacco callus lines, but no correlation between copy number and expression level could be found (Allen *et al*., 1993). In contrast, the MAR element from the soybean heat shock gene *Gmhsp17.6-L* was shown to be capable of increasing expression levels but had little effect on variability (Schöffl *et al*., 1993). A soybean MAR flanking a reporter gene construct reduced variability of expression when compared to a construct lacking MARs, but also reduced expression levels when present 5' and 3' of a reporter gene construct in transgenic tobacco callus (Breyne *et al*., 1992). It is possible that individual MARs can have different functional and structural properties in addition to their matrix binding ability (Breyne *et al*., 1994).

MARs are usually 300 to 2000 base pairs in length, are rich in adenosine and thymine residues and often contain certain conserved sequence elements and structural features. Most MARs described in the literature are not obtained from plants, but it has been well documented that MARs from other organisms bind plant scaffolds and vice versa (Dietz *et al*., 1994; Breyne *et al*., 1992).

Table 1 describes sequence elements present in MARs described in the literature, including the following plant MARs: soybean heat shock protein gene MAR, (Schöffl *et al*., 1993); a petunia MAR (Dietz *et al*., 1994); the pea plastocyanin gene MAR (Slatter *et al*., 1991); the maize Adh1 gene 5' and 3' MARs (Avramova and Bennetzen, 1993; Avramova *et al*., 1995); the b-phaseolin gene 5' and 3' MARs (van der Geest *et al*., 1994.

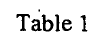

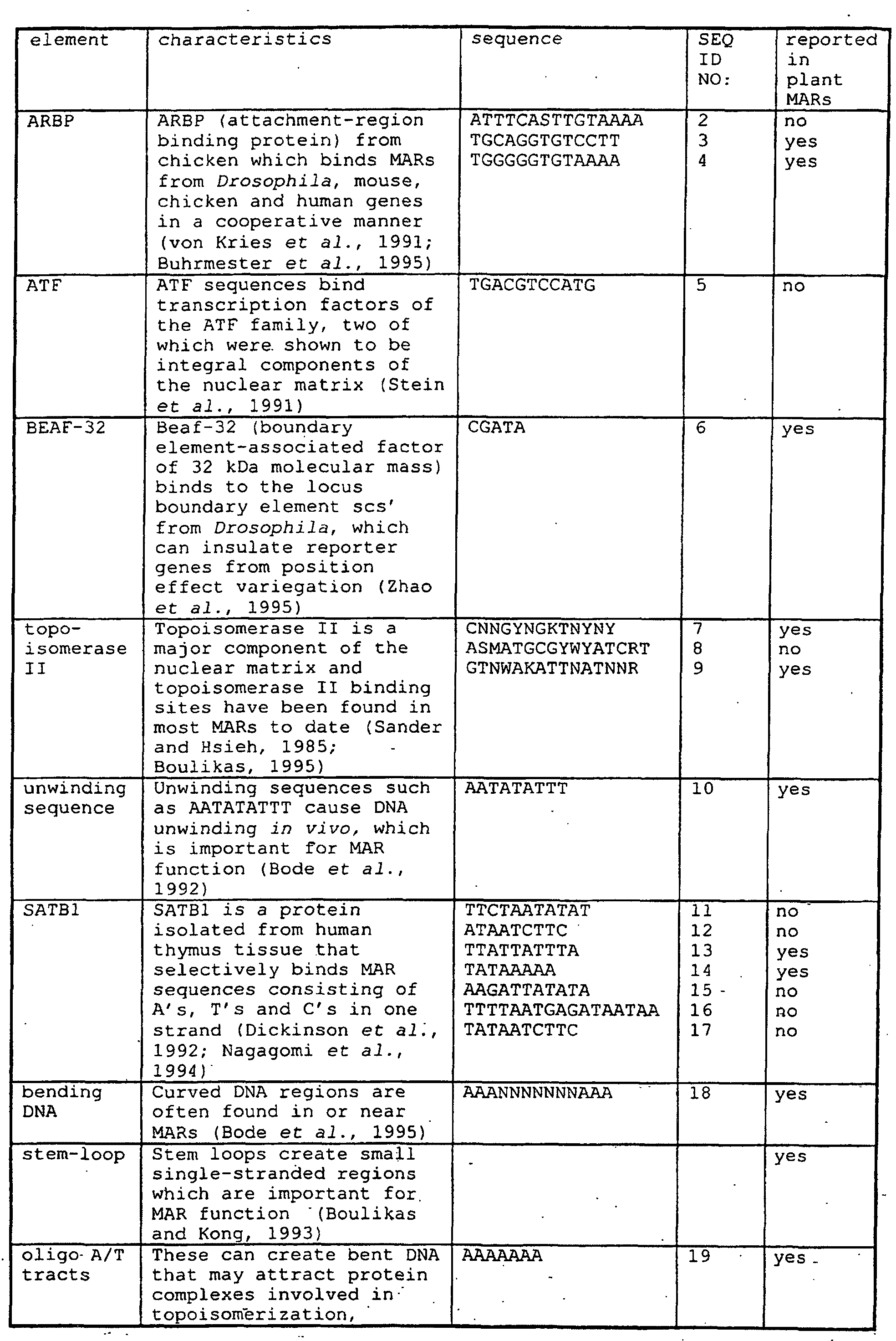

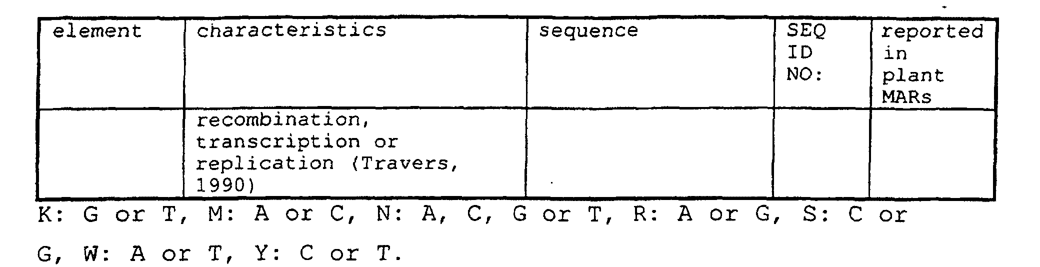

The present invention utilizes a subset of the features described in Table 1 in a novel artificial MAR. The sequence of the 327 bp artificial MAR is given in SEQ ID NO:1. The artificial MAR was designed as a sequence flanked by *Bgl*II and *Bam*HI restriction sites, which are included in SEQ ID NO:1, but which are not critical to the function of the MAR. The functional portion of the MAR comprises bp 11 to 309 of SEQ ID NO:1.

The following features are found in SEQ ID NO:1:

| Feature | location (bp) |
|---|---|
| *Bgl*II | 5-10 |
| BEAF-32 | 11-15 |
| SATBI | 24-34 |
| unwinding | 28-36 |
| topoisomerase II | 44-59 |
| ATF site | 60-69 |
| A/T tract | 70-85 |
| BEAF-32 | 86-90 |
| stem-loop | 93-101/117-124 |
| unwinding | 105-113 |
| topoisomerase II | 125-139 |
| SATBI | 149-159 |
| unwinding | 153-161 |
| BEAF-32 | 164-168 |
| SATBI | 185-195 |
| stem-loop | 208-216/231-239 |
| unwinding | 219-227 |
| A/T tract | 241-253 |
| topoisomerase II | 268-283 |
| curved (bending) DNA | 284-294 |
| ARBP site | 295-309 |
| BamHI | 318-323 |

The 3' UTR, or 3' untranslated region, that is employed in constructs of the invention is one that confers efficient processing of the mRNA, maintains stability of the message and directs the addition of adenosine ribonucleotides to the 3' end of the transcribed mRNA sequence. The 3' UTR may be native with the promoter region, native with the structural gene, or may be derived from another source. Suitable 3' UTRs include, but are not limited to: the *per5* 3' UTR, and the 3' UTR of the nopaline synthase (*nos*) gene.

### Example 1

### Synthesis of artificial MAR

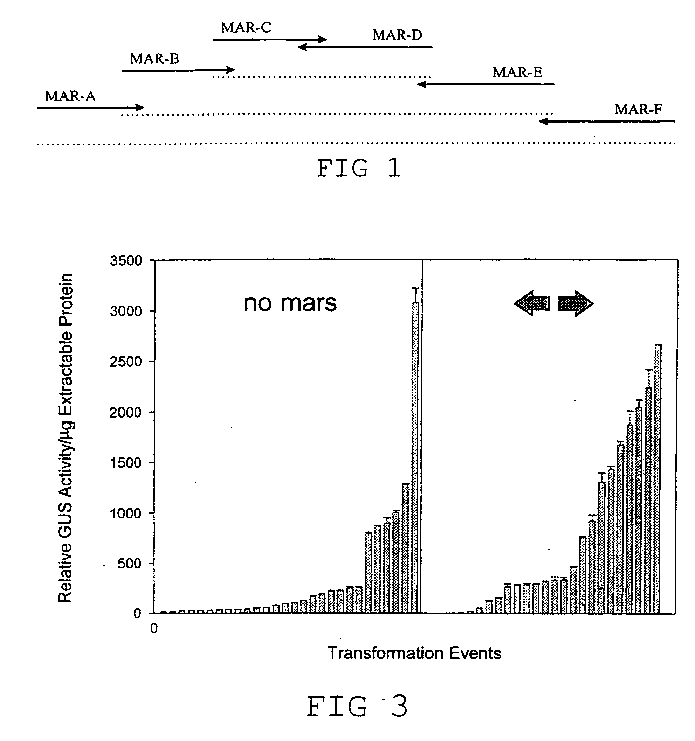

To construct the artificial MAR, six individual oligonucleotides were synthesized and assembled by PCR. The sequences for the six oligonucleotides, referred to hereinafter as MAR-A, MAR-B, MAR-C, MAR-D, MAR-E, and MAR-F, are given in the Sequence Listing as SEQ ID NOS: 20 through 25, respectively. A 15 bp overlap between adjacent oligonucleotides allowed assembly of the MAR by PCR, using the strategy shown in Figure 1.

The GeneAmp™ PCR Reagent Kit with AmpliTaq DNA Polymerase (Perkin Elmer, Norwalk, CT) was used for the DNA amplification. Twenty cycles of PCR (denaturation: 30 sec at 94°C; annealing: 60 sec at 52°C and extension: 60 sec at 70°C) with primers MAR-C and MAR-D were followed by 20 cycles of PCR with primers MAR-B and MAR-E, using the product from the first reaction as template for the second reaction. The 231 bp product of this reaction was purified from a low melting point agarose gel and used as a template for 20 cycles of PCR with primers MAR-A and MAR-F. The 327 bp product from this reaction was subcloned into pCR2.1 using the TA Cloning™ Kit (Invitrogen, San Diego, CA) and the sequence was verified by sequencing.

A dimer consisting of two tandem copies of the *Bgl*II/*Bam*HI fragment was constructed in the *Bam*HI site of pBluescript™ SK- (Stratagene, La Jolla, CA). The sequence of the dimer is bp 5-630 of SEQ ID NO:26.

### EXAMPLE 2

### Binding of artificial MAR to nuclear scaffolds

### A. Controls

Two DNA fragments of similar size and nucleotide composition as the artificial MAR were amplified from plant DNA to serve as controls in the binding assay. These fragments were a 657 bp fragment from the 3' end of a maize gene Gpal (glyceraldehyde-3-phosphate dehydrogenase subunit A, GenBank accession number X15408, bases 4516 to 5173, Quigley *et al.*, 1989), and a 488 bp fragment from the 5' flanking region of a 19 kD alpha zein gene (GenBank accession number X05911, bases 339 to 827, Kriz *et al*., 1987). Table 2 compares the features present in the artificial MAR and control fragments.

**Table 2**

| element | Artificial MAR dimer | Gpal control | zein control |
|---|---|---|---|
| ARBP sites | 2 | 0 | 0 |
| ATF sites | 2 | 0 | 0 |
| BEAF-32 sites | 6 | 1 | 1 |
| topoisomerase II sites | 6 | 0 | 0 |
| unwinding sequence sites | 8 | 0 | 0 |
| SATB1 sites | 6 | 0 | 0 |
| bending DNA sites | yes | yes | yes |
| stem-loop sites | 4 | 1 | 0 |
| oligo A/T tracts | 2 | 0 | 1 |
| fragment size in binding assay | 632 | 657 | 488 |
| % A + T | 71 | 63 | 66 |
| strength of binding to nuclear scaffolds | +++++ | - | - |

### B. Preparation of nuclei from maize leaves

Nuclei for use in isolating nuclear scaffolds were prepared from young maize leaves by adaptation of a published protocol (Hall *et al*., 1991). Nuclei were counted and checked for integrity by microscopic examination of DAPI stained aliquots. Only high quality nuclei were used to prepare nuclear scaffolds by lithium diiodosalicylate extraction.

For nuclei purification, young maize leaves from V4 stage plants (fourth or fifth leaf) were harvested with a razor blade, washed and dried. After removing the midrib, leaves were frozen in liquid nitrogen, and ground to a fine powder with a mortar and pestle. The powdered leaf samples were transferred to a glass beaker, and 5 ml NIB1+PI (0.5 M hexylene glycol, 20 mM piperazine-N,N'-bis[2-ethanesulfonic acid] (PIPES), pH 6.5, 20 mM KCl, 7 mM 2-mercaptoethanol, 0.5 mM ethylenediaminetetraacetic acid (EDTA), 0.4 % Triton X-100™ [Rohm & Haas Company, Philadelphia, PA], 0.05 mM spermine, 0.125 mM spermidine, 1 mM phenylmethylsulfonyl fluoride (PMSF), 1 µg/ml leupeptin, 1 µg/ml aprotinin) was added per gram of leaf tissue. The leaf extract was filtered sequentially through 1900, 520, 125, 85 and 40 mm filters at 4°C and filters were rinsed with 1 ml NIB1+PI per gram leaf to collect any nuclei that were trapped in the debris. Fifteen ml crude nuclear extract was loaded onto Percoll™ (Pharmacia Biotech, Piscataway, NJ) gradients consisting of 7 ml 40% Percoll in NIB1 (0.5 M hexylene glycol, 20 mM PIPES, pH 6.5, 20 mM KCl, 7 mM 2-mercaptoethanol, 0.5 mM EDTA, 0.4 % Triton™ X-100, 0.05 mM spermine, 0.125 mM spermidine) and 5 ml 70% Percoll in NIB1. After centrifugation for 15 min at 500xg at 4°C the 40%/70% interface was collected with a sterile pasteur pipette and added to 2 volumes NIB2 (0.5 M hexylene glycol, 20 mM PIPES, pH 6.5, 20 mM KCl, 7 mM 2-mercaptoethanol, 0.5 mM EDTA, 0.05 mM spermine, 0.125 mM spermidine), taking care to avoid the pellet and other debris. Nuclei were concentrated by centrifugation at 600x*g* for 10 min at 4°C.

The nuclear pellet was resuspended in 20 ml NIB2 and centrifuged as before. This step was repeated one more time to wash away traces of Percoll. Nuclei were counted using a hemacytometer and resuspended in NIB2+PI/50% glycerol (0.5 M hexylene glycol, 20 mM PIPES, pH 6.5, 20 mM KCl, 7 mM 2-mercaptoethanol, 0.5 mM EDTA, 0.05 mM spermine, 0.125 mM spermidine, 50% glycerol, 1 mM PMSF, 1 µg/ml leupeptin, 1 µg/ml aprotinin) at 20 million nuclei/ml. Nuclei were stored at -80°C until used for scaffold preparation.

### C. Preparation of nuclear scaffolds

Frozen nuclei were thawed and washed with 10 ml of NIB3+PI (0.5 M hexylene glycol, 20 mM PIPES, pH 6.5, 20 mM KCl, 7 mM 2-mercaptoethanol, 0.05 mM spermine, 0.125 mM spermidine, 1 mM PMSF, 1 µg/ml leupeptin, 1 µg/ml aprotinin) per 20 million nuclei. Nuclei were collected by centrifugation at 600xg for 10 minutes, resuspended in 200 ml NIB3+PI in the presence of 1 mM CuSO₄, and incubated for 10 min at 42°C to stabilize the nuclei.

Histones were extracted by incubation in 10 ml HIB+PI (20 mM HEPES, pH 7.4, 100 mM lithium acetate, 10 mM LIS (lithium diiodosalicylate), 0.1 % digitonin, 2 mM EDTA, 1 mM PMSF, 1 µg/ml leupeptin, 1 µg/ml aprotinin) for 15 minutes at room temperature. The resulting nuclear halos were transferred to a centrifuge tube and pelleted at 4000x*g* for 10 minutes. Halos were washed twice with 10 ml HWB (20 mM Tris, pH 8, 70 mM NaCl, 20 mM KCl, 7 mM 2-mercaptoethanol, 0.1 % digitonin, 0.05 mM spermine, 0.125 mM spermidine) and once with D/BB+PI (HWB + 10 mM MgCl₂, 1 mM PMSF, 1 µg/ml leupeptin, 1 µg/ml aprotinin) to remove LIS. If halos did not pellet well, subsequent centrifugation steps were done at 6000xg using a slow brake setting. The quality of the halos was verified by SDS-PAGE gel to ensure that more than 95% of the histones were removed in the extraction procedure

Washed nuclear halos were resuspended in 400 µl D/BB+PI and 200 units of restriction enzymes (100 u each of *Eco*RI and *Hind*III) were added, and incubated at 37°C for 2-3 hours on a rocking platform to keep the halos from settling. The restriction enzymes removed more than 70% of the nuclear DNA, producing nuclear scaffolds. Scaffolds were pelleted at 300xg and washed with HWB+PI (HWB + 1 mM PMSF, 1 µg/ml leupeptin, 1 µg/ml aprotinin). Nuclear scaffolds were resuspended in 400 µl HWB+PI and separated into 100 µl aliquots (containing 5 million nuclear equivalents).

### D. Binding of artificial MAR to nuclear scaffolds

100 µl aliquots of scaffolds in HWB+PI were incubated with probe and *E. coli* competitor DNA at 37°C for 2-3 hours in siliconized microfuge tubes on a rocking platform shaker. After incubation, the supernatant fraction (containing unbound DNA fragments) and pellet fraction (containing scaffolds and bound DNA fragments) were separated via centrifugation in a horizontal microfuge at 3000xg for 5 min. The pellet was washed once with 200 µl HWB to remove proteinase inhibitors, resuspended in 100 µl lysis buffer (10 mM Tris, pH 8, 10 mM EDTA, 0.5 % SDS, 0.5 mg/ml Proteinase K) and incubated overnight at room temperature.

Equal fractions of the pellet and supernatant were separated on a 0.9% agarose gel, which was subsequently fixed, by soaking in 7% TCA for 20 min, dried and exposed to X-ray film at room temperature and/or storage phosphor screens for the PhosphoImager™ SI (Molecular Dynamics, Sunnyvale, CA).

Plasmids containing the artificial MAR monomer or dimer or the control Gpal or zein sequences were digested with restriction enzymes that generate 5' overhang ends. The Klenow subunit of DNA polymerase I was used to fill the overhang with [a-³²P]dCTP (Amersham Life Science, Arlington Heights, IL). The end-labeled DNA fragments were used as probes in the binding assay, i.e. the fragments were incubated with purified maize nuclear scaffolds in the presence of unlabeled *E.coli* competitor DNA and the relative binding of the inserts was determined. Relative amounts of nuclei, probe and unlabeled *E. coli* competitor DNA used in the binding assay were optimized to obtain maximal discrimination between strongly and weakly binding MARs. The optimal relative amounts were 2, 5 or 10 µg of unlabeled *E. coli* competitor DNA, 5 million nuclear equivalents of nuclear scaffolds, and 1 fmole of digested and labeled plasmid per assay.

The artificial MAR dimer bound very strongly to the nuclear scaffold preparation, even in the presence of high levels of competitor DNA. The monomer MAR also bound to nuclear scaffold preparations, albeit at a lower affinity. Neither control sequence was retained in the pellet fraction, even though they were similar to the artificial MARs in size and relative AT content. This suggests that the elements included in the artificial MAR facilitate binding.

### EXAMPLE 3

### EVALUATION OF THE ARTIFICIAL MAR IN RICE

### A. Rice Transformation Vectors

pGOS2-hpt (SEQ-ID NO:27) is a rice transformation vector containing a hygromycin selectable marker driven by the 35S promoter and a GOS2/GUS/nos cassette (GOS2 transcription initiation region/GUS structural gene/nos 3' untranslated region). The GOS2 transcription inititation region in this construct is comprised of 1010 bp of promoter and 170 bp of untranslated 5' leader interrupted by a 1100 bp intron (de Pater *et al*., 1992).

pArGOS2Af-hpt (SEQ ID NO:28) is a rice transformation vector identical to pGOS2-hpt except that it has the MAR dimer of SEQ ID NO:29 positioned 5' to the GOS2 transcription initiation region and the MAR dimer of SEQ ID NO:26 positioned 3' to the nos 3' UTR.

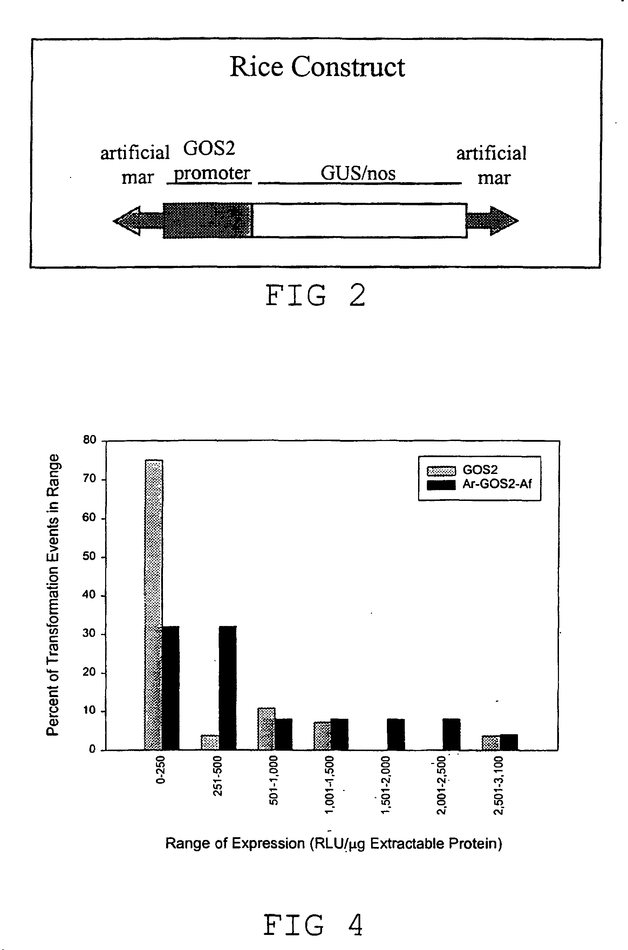

A schematic representation of the ArGOS2Af construct is shown in FIG 2.

### B. Transformation of Rice

For initiation of embryogenic callus, mature seeds of a *Japonica* cultivar, Taipei 309, were dehusked and surface-sterilized in 70% ethanol for 5-7 min. followed by soaking 30-45 min in 25% commercial bleach (2.6% sodium hypochlorite) with 0.02% Tween™ 20 (ICI Americas, Inc.) under vacuum. The seeds were then rinsed 5 times in sterile distilled water and placed on filter paper before transferring to induction media (NB). The NB medium consisted of N6 macro elements (Chu, 1978), B5 micro elements and vitamins (Gamborg *et al.*, 1968), 300 mg/l casein hydrolysate, 500 mg/l L-proline, 500 mg/l L-glutamine, 30 g/l sucrose, 2 mg/l 2-,4-dichloro-phenoxyacetic acid (2,4-D), and 2.5 g/l Gelrite™ (Merck & Co., Rawhay, NJ) with the pH adjusted to 5.8. The mature seed cultured on induction media were incubated in the dark at 28° C for three weeks. Primary callus induced from the scutellar region of mature embryo was transferred to fresh NB medium for further maintenance and thereafter maintained on a two week subculture period.

To prepare DNA for blasting, about 140 µg of plasmid DNA (pGOS2-hpt or pArGOSAf-hpt) was precipitated onto 60 mg of gold particles. The plasmid DNA was precipitated onto 1.5-3.0 micron (Aldrich Chemical Co., Milwaukee, WI) or 1.0 micron gold particles (Bio-Rad Laboratories, Hercules, CA). The precipitation mixture included 60 mg of pre-washed gold particles, 300 µl of water/DNA (140 µg), 74 µl of 2.5 M CaCl₂, and 30 µl of 0.1 M spermidine. After adding the components in the above order, the mixture was vortexed immediately, and allowed to settle for 2-3 min. The supernatant was pipetted off and discarded. The DNA-coated gold particles were resuspended in 1 ml of 100% ethanol and diluted to 17.5 mg DNA/7.5 mg gold per ml of ethanol for use in blasting experiments.

For helium blasting, actively growing embryogenic callus cultures, 2-4 mm in size, were subjected to a high osmoticum treatment by placing callus on NB medium with 0.2 M mannitol and 0.2 M sorbitol (Vain *et al*., 1993) for 4 hr before helium blasting. Following osmoticum treatment, callus cultures were transferred to blasting medium (NB+2% agar) and covered with a stainless steel screen (230 micron). Helium blasting involved accelerating the suspended DNA-coated gold particles towards and into the prepared tissue targets. The device used was an earlier prototype to the one described in US Patent No. 5,141,131, which is incorporated herein by reference, although both function in a similar manner. The callus cultures were blasted at different helium pressures (1750-2,250 psi) one to three times per target. After blasting, callus was transferred back to the high osmotic media overnight before placing on selection medium, which consisted of NB medium with 30 mg/l hygromycin. After 2 weeks, the cultures were transferred to fresh selection medium with higher concentrations of selection agent, i.e., NB+50 mg/l hygromycin (Li. *et al.,* 1993).

Compact, white-yellow, embryogenic callus cultures, recovered on NB+50 mg/l hygromycin, were regenerated by transferring to pre-regeneration (PR) medium+50 mg/l hygromycin. PR medium consisted of NB medium with 2 mg/l benzyl aminopurine (BAP), 1 mg/l naphthalene acetic acid (NAA), and 5 mg/l abscisic acid (ABA). After 2 weeks of culture in the dark,.they were transferred to regeneration (RN) medium. The composition of RN medium is NB medium with 3 mg/l BAP, and 0.5 mg/l NAA. The cultures on RN medium were incubated for 2 weeks at 28° C under high fluorescent light (325-ft-candles). The plantlets with 2 cm shoots were transferred to 1/2 MS medium (Murashige and Skoog, 1962) with 1/2 B5 vitamins, 10 g/l sucrose, 0.05 mg/l NAA, 50 mg/l hygromycin and 2.5 g/l Gelrite™ adjusted to pH 5.8 in GA7 vessels (Magenta Corp., Chicago, IL). When plantlets were established with well-developed root systems, they were transferred to soil [1 part Metro-Mix 360 (Scotts-Sierra Horticultural Products Co., Marysville, OH) and 1 part top soil) and raised in a growth chamber (29/24°C day/night cycle, 50-60% humidity, 12 h photoperiod) until they reached a height of 60 cm, at which point 2 leaves were harvested for quantitative GUS analysis, and the plants were transferred to the greenhouse to grow to maturity.

### C. Southern analyses

Southern analysis was used to identify primary regenerate (R₀) rice lines lines that contained intact copies of the specific gene construct.

A DNA probe specific for the coding region of the β-glucuronidase (GUS) gene construct was gel purified with the Qiaex II DNA purification kit (Qiagen Inc., Chatsworth, CA). Radiolabeled probe was prepared using the Ready-To-Go™ DNA labeling beads (Pharmacia LKB, Piscataway, NJ) with 50 microcuries of [α³²P]dCTP (Amersham Life Science, Arlington Heights, IL).

Leaf material from R₀ rice plants was harvested from two representatives from each line. Genomic DNA from the R₀ plants was prepared from lyophilized tissue as described by Saghai-Maroof *et al*. (1984).

Four micrograms of rice DNA was digested with restriction enzyme to release the intact gene construct using conditions suggested by the manufacturer (Bethesda Research Laboratory, Gaithersburg, MD) and separated by agarose gel electrophoresis. The DNA was blotted onto nylon membranes as described by Southern (1975, 1989). Radiolabeled probe DNA .was hybridized to the genomic DNA on the blots using 50 ml of minimal hybridization buffer (10% polyethylene glycol, 7% sodium dodecyl sulfate, 0.6x SSC, 10 mM sodium phosphate, 5 mM EDTA and 100 mg/ml denatured salmon sperm DNA) heated to 60°C and mixed with the denatured radiolabeled probed prior to being added to the blots for overnight hybridization at 60°C. The blots were washed at 60°C in 0.25X SSC and 0.2% SDS for 45 minutes, blotted dry and exposed to XAR-5 film with two intensifying screens overnight.

Southern analysis was conducted on seventy ArGOS2Af R₀ rice lines. The DNA from the R₀ plants was digested with the restriction enzyme XbaI which, if the intact gene construct is present, should result in a 5.7 kb hybridization product when radiolabeled with a probe specific for the GUS coding region. The 5.7 kb fragment should consist of the artificial MAR in the reverse orientation, the GOS2 promoter, the GUS coding region, the nos 3' UTR and the artificial MAR in the forward orientation. The expected 5.7 kb hybridization product was detected in twenty-five of the seventy rice lines. All of the twenty-five lines had multiple hybridization products and two of the lines had identical complex hybridization patterns indicating that they are probably from the same transformation event.

The non-Mar control lines, GOS2, were also analyzed by Southern analysis. The DNA from forty-eight GOS2 R₀ lines was digested with the restriction enzymes *Eco*RI and *Xba*I which, if the intact gene is present, should result in a 4.4 kb hybridization product when radiolabeled with a probe specific for the GUS coding region. The 4.4 kb fragment would include 1.6 kb of the GOS2 promoter, the GUS coding region, the nos 3' UTR and the 35T promoter (the promoter used to drive the selectable marker gene). The expected 4.4 kb hybridization product was detected in twenty-eight of the forty-eight GOS2 lines. Two of the lines had identical hybridization patterns and must have resulted from'the same-transformation event. Two of the lines contained genetic chimeras.

### D. GUS analysis

Analysis of rice was performed on young leaves of primary transformants, after plants had been grown 6-8 weeks in an environmentally controlled growth chamber and had reached a height of about 60 cm. Two independently regenerated rice plants were analyzed per transformation event. Individual transformants were analyzed by Southern blots to verify the presence of an intact copy of the transgene and determine whether each event displayed unique hybridization patterns, indicating independent transformation events. Plants lacking a complete copy of the transgene, chimeric events, or duplicated integration events were not included in the analysis.

Results of the analysis are reported in FIGS 3 and 4. In FIG 3, error bars represent the standard deviation between the plants for each transformation event. Two samples were independently processed for each plant. In general, the level of expression of GUS in independent rice plants from each transformation event was similar (as demonstrated by the standard deviation of the results shown in FIG 3).

FIG 4 reports the percent of transformation events expressing GUS in the indicated ranges.

### EVALUATION OF ARTIFICIAL MAR IN ARABIDOPSIS

### A. Arabidopsis Transformation Vectors

Act2/GUS/nos (Act2 transcription initiation region/GUS structural gene/nos 3' UTR) constructs were made for testing in a dicot system (*Arabidopsis*). Three vectors were made:
pAct2-bin (SEQ ID NO:30) is a binary vector containing a Act2/GUS/nos cassette, 19S/NPTII/orf25polyA as a selectable marker, and 35S/GFP/nos as an independent reporter gene.
pArAct2Af-bin(SEQ ID NO:31) is identical to pAct2-bin except that it has the MAR dimer of SEQ ID NO:29 positioned 5' to the Act2 transcription initiation region, and the MAR dimer of SEQ ID NO:26 positioned 3' to the nos 3' UTR.
pAfAct2Af-bin (SEQ ID NO:32) is identical to pAct2-bin except that it has the MAR dimer of SEQ ID NO:26 positioned 5' to the Act2 transcription initiation region the MAR dimer of SEQ ID NO: 33 positioned 3' to the nos 3' UTR.

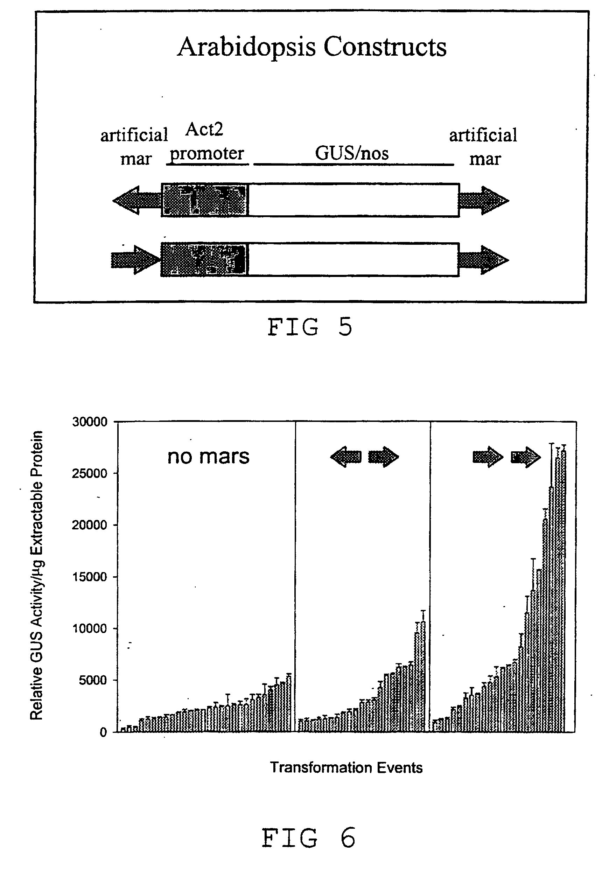

These vectors enabled testing of two orientations of the artificial MAR dimer in *Arabidopsis*. A schematic of the pArAct2Af-bin and pAfAct2Af-bin constructs is shown in Figure 5.

### B. Arabidopsis transformation

*Arabidopsis* transformation was performed according to a protocol provided by Pam Green (van Hoof and Green 1996), which is an adaptation from protocols by Nicole Bechtold (Bechtold *et al*., 1993), Andrew Bent (Bent *et al*., 1994) and Takashi Araki (personal communication).

Seeds of ecotype Columbia were planted in 4 inch square pots, covered with window screen mesh, and grown under conditions of 16 hours light/ 8 hours dark at 22°C, fertilizing by subirrigation once a week. The fertilizer consisted of 5 mM KNO₃, 2.5 mM KPO₄ (pH 5.5), 2 mM MgSO₄, 2 mM Ca(NO₃)₂, 0.05 mM Fe•EDTA, 0.07 mM boric acid, 0.014 mM MnCl₂, 0.005 mM CuSO4, 0.001 mM ZnSO₄ 0.0002 mM NaMoO₄, and 0.01 mM NaCl. Plants were thinned to 4 plants per pot and grown until several bolts emerged. When plants were ready to transform, the above soil parts were submerged in infiltration medium (2.2 g/l MS salts, 1X B5 vitamins, 50 g/l sucrose, 2.5 mM MES, pH 5.7, 0.044 M benzylaminopurine, 200 ml/l Silwet L-77™ [Osi Specialties, Inc.] containing *Agrobacterium* cells, placed inside a vacuum desiccator under a vacuum of 400 mm Hg (about 17 inches) for 5 minutes. After quickly releasing the vacuum, pots were drained and placed on their sides in a tray covered with plastic wrap to maintain humidity for 24 hours. The next day the pots were uncovered and set upright. Plants were staked individually and after 2 weeks watering was gradually reduced to allow plants to dry out. Seeds were harvested from each plant individually.

For selection of transformation events, 1-10 mg seeds per plant were surface sterilized by soaking in 10% bleach for 7 minutes while mixing vigorously, followed by three rinses in sterile water, and placed in a flask containing *Arabidopsis* germination medium (MS salts, MS vitamins, 10% sucrose, 2.5 mM 2-[N-morpholino]ethanesulfonic acid [MES], 30 mg/l kanamycin, 50 mg/l vancomycin and 0.1 % Bacto™-Agar [Difco Laboratories, Detroit, MI]). After shaking in continuous light at 90 rpm for 3 days, seeds germinated, and transformants were isolated as green seedlings between 7 and 12 days after germination. Nontransformed seeds produced small bleached seedlings. Transformants were transferred to solid medium (MS salts, B5 vitamins, 10% sucrose, 2.5 mM MES, 15 g/l Phytagar™ [Gibco BRL, Gaithersburg, MD], 30 ml/l kanamycin, 50 mg/l vancomycin) in plates for further selection. After one to two weeks, true transformants were transferred to GA7 vessels (MS salts, B5 vitamins, 0.3 % sucrose, 2.5 mM MES) for one to two weeks prior to planting in soil for production of T1 seed.

### C. Southern analyses

Southern analysis was used to identify primary regenerate T2 *Arabidopsis* lines that contained intact copies of the specific gene construct.

Pooled samples of *Arabidopsis* leaf tissue were powdered in liquid nitrogen. The ground tissue was then incubated for three minutes in 500 µl 2X extraction buffer (2% CTAB, 100 mM Tris-HCl, pH 8.0, 20 mM EDTA, 1.4 M NaCl and 2% 2-mercaptoethanol) at 65°C. Five hundred µl of chloroform/octanol (24:1) was added, the samples were shaken for two minutes, and then spun at 14,000 ' g in a microcentrifuge and the supernatant was removed. The chloroform/octanol extraction was repeated. One ml of precipitation buffer (1% CTAB, 50 mM Tris-HCl, pH 8.0, 10 mM EDTA, and 1% 2-mercaptoethanol) was added to the supernatant and then incubated at room temperature for 60 minutes. The DNA was pelleted by centrifugation at 3500g for 5 minutes in a microcentrifuge. The pellet was drained and resuspended in 200 µl 1.0 M NH₄OAc. One hundred µl 7.5 M NH₄OAc and 1 ml isopropanol were added, the samples incubated on ice for 5 minutes and then centrifuged at 14,000 g for 5 minutes. The pellet was drained and resuspended in 200 µl TE. 100 µl 7.5 M NH₄OAc and 1 ml isopropanol were added and incubated on ice for 5 minutes then centrifuged at 14,000 g for 5 minutes. The pellet was drained and rinsed with 70% ethanol and dried in a Speed Vac (Savant Instruments Inc., Farmingdale, NY). The dried pellet was resuspended in 20 µl TE (10 mM TRIS, 1 mM EDTA, pH 8.0).

Southern analysis was conducted on 29 ArAct2Af T2 lines and 24 AfAct2Af T2 lines. One microgram of DNA from the ArAct2Af plants was digested with the restriction enzyme *Xba*I using conditions suggested by the manufacturer (Bethesda Research Laboratory, Gaithersburg, MD) and separated by agarose gel electrophoresis, which should result in a 4.6 kb hybridization product, if the gene construct is intact, when radiolabeled with a probe specific for the GUS coding region. Similar to the ArGOS2Af rice plants, the 4.6 kb fragment should consist of the artificial MAR in the reverse orientation, the Act2 promoter, the GUS coding region, the nos 3' UTR and the artificial MAR in the forward orientation. The DNA was blotted onto nylon membranes as described by Southern (1975, 1989). Radiolabeled probe DNA was hybridized to the genomic DNA on the blots using 50 ml of minimal hybridization buffer (10% polyethylene glycol, 7% sodium dodecyl sulfate, 0.6x SSC, 10 mM sodium phosphate, 5 mM EDTA and 100 mg/ml denatured salmon sperm DNA) was heated to 60°C and mixed with the denatured radiolabeled probed prior to being added to the blots for overnight hybridization at 60°C. The blots were washed at 60°C in 0.25X SSC and 0.2% SDS for 45 minutes, blotted dry and exposed to XAR-5 film with two intensifying screens overnight.

The expected 4.6 kb hybridization product was detected in twenty-six of the twenty-nine ArAct2Af lines. A second Southern blot was generated to determine the copy number of the ArAct2Af construct. The ArAct2Af DNA was digested with the restriction enzymes *Sst*I and *Xho*I which cut the construct near the right and left borders of the T DNA. The blots were radiolabeled with probes specific for the artificial MAR and the DNA from the left border to the *Xho*I site 800 bp downstream. A single copy of the ArAct2Af construct will have three hybridization products: two fragments of unknown size, consisting of the left and right border DNA, and the 8.9 kb fragment which is the DNA internal to the borders. Twenty-two of the twenty-nine lines had three or fewer hybridization products, indicating that a single copy of the ArAct2Af construct was present.

The DNA from the AfAct2Af plants was digested with the restriction enzyme *Xba*I which, if the construct remains intact, should result in a 5.7 kb hybridization product when radiolabeled with a probe specific for the GUS coding region. The 5.7 kb fragment should consist of the artificial MAR in the forward orientation, the Act2 promoter, the GUS coding region, the nos 3' UTR and the artificial MAR in the forward orientation. It also includes the green fluorescent protein coding region and the nos 3' UTR. The expected 5.7 kb hybridization product was detected in all of the twenty-four AfAct2Af lines.

A second Southern blot was generated to determine the copy number of the AfAct2Af construct. The AfAct2Af DNA was digested with the restriction enzymes *Sst*I and *Xho*I which cut the construct near the right and left borders of the T DNA. The blots were radiolabeled with probes specific for the artificial MAR and the DNA from the left border to the *Xho*I site 800 bp downstream. A single copy of the AfAct2Af construct will have three hybridization products: two fragments of unknown size, consisting of the left and right border DNA and the 8.9 kb fragment which is the DNA internal to the borders. Fifteen of the twenty-four lines had three or fewer hybridization products indicating that a single copy of the AfAct2Af construct was present.

The non-Mar control lines, Actbin, were also analyzed by Southern analysis. The DNA from thirty-four Actbin T2 lines was digested with the restriction enzyme PstI which should, if the construct remains intact, result in a 3.4 kb hybridization product when radiolabeled with a probe specific for the GUS coding region. The 3.4 kb fragment should consist of the Act2 promoter, the GUS coding region, and the nos 3' UTR. The expected 3.4 kb hybridization product was detected in thirty of the thirty-four Actbin lines. A second Southern blot was generated to determine the copy number of the Actbin construct. The DNA from the Actbin lines was digested with the restriction enzymes *Sst*I and *Xho*I which cut the construct near the right and left borders of the T DNA. The blots were radiolabeled with probes specific for the nos 3'UTR and the DNA from the left border to the *Xho*I site 800 bp downstream. A single copy of the Actbin construct will have three hybridization products: two fragments of unknown size, consisting of the left and right border DNA and the 8.2 kb fragment which is the DNA internal to the borders. Nine of the thirty-four lines had three or fewer hybridization products indicating that a single copy of the Actbin construct was present.

### D. GUS analysis

For growing *Arabidopsis*, T2 seed was germinated *in vitro* on MS medium containing 90 mg/l kanamycin and 3 week old kanamycin resistant seedlings were harvested. Two batches of 30 seedlings per transformation event were used for GUS analysis and additional seedlings were used to extract DNA.

For analyses of GUS activity, leaf samples were powdered in liquid nitrogen and samples of approximately 400 ml of tissue were placed in microfuge tubes. Two independent samples from each leaf sample was processed. The tissue was either stored at -70°C or extracted immediately. GUS was extracted by mixing the powdered tissue with GUS lysis buffer (Jefferson *et al*., 1987) modified by the addition of 1% polyvinylpolypyrrolidone (hydrated in the buffer for at least one hour) and 20% glycerol. After incubation on ice for at least 10 min, the samples were centrifuged at 16,000 g for 10 min. The supernatants were recovered and centrifuged a second time as described above. The supernatants were recovered and frozen on dry ice and stored at -70°C. Experiments showed that GUS activity was stable for at least 4 freeze-thaw cycles when stored in the buffer described above (W.M. Ainley, unpublished). GUS activity was measured using a GUS-Light™ kit (Tropix, Inc., Bedford, MA). Five ml samples of undiluted extract or of extract diluted so that the luminescence was within the range measured by the luminometer was added to 195 ml of the GUS-Light™ Reaction Buffer. Luminescence was integrated for 5 sec after a 5 sec delay. Protein was measured with the assay developed by Bradford (1976) using human serum albumin as the standard. GUS activity was normalized between experiments using a GUS standard obtained from SIGMA. The amount of plant protein in the standards and plant samples was the same; protein was adjusted where necessary using extracts of nontransformed plants.

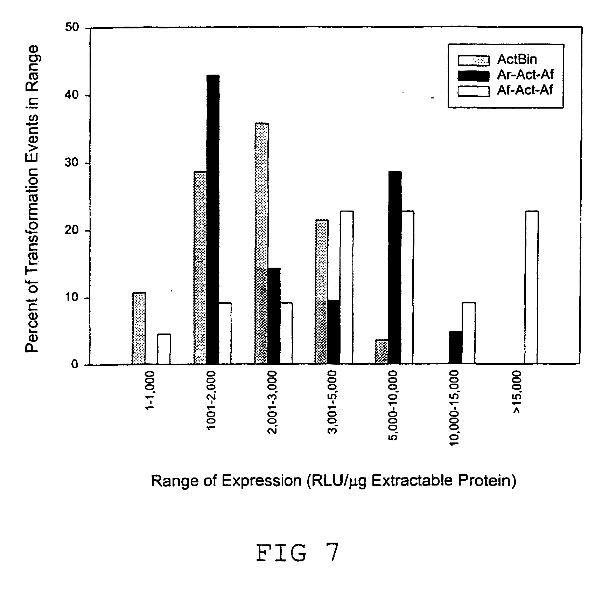

FIGS 6 and 7 report the results the GUS analysis. FIG 6 reports expression observed for independent transformation events expressing either the base, non-MAR construct (Actbin) or the base construct flanked by the artificial MAR in the indicated orientation (ArAct2Af or Af-Act-Af). Standard deviations indicate the variance between plants harvested from different plates. Arrows indicate the relative orientations of the MARs. E.

In FIG 7, percent of transformation events expressing GUS in the indicated ranges is shown.

### E. Characterization of transgenic plants expressing the reporter gene constructs

T2 *Arabidopsis* plants were analyzed. The plants were evaluated for segregation of kanamycin resistance to determine insert copy number. A few events did not fall into either one or two insert categories as determined by chi square analyses. Of the remaining plants, 72% had a single insert. Most of the inserts had multiple copies of the transgene. Although the Arabidopsis transformation events were grown under controlled environments, there were some occasional differences in the relative growth of plants between the duplicate plates. In those cases, the events were either not used or were grown again. The coefficient of variance of the expression determined from duplicate plates range of between 1 and 46%, with 89 percent below 20 percent (Figure 6).

Generally, all *Arabidopsis* transformation events tested for a construct were grown and analyzed together. Although the growth of the plants was under controlled environments, the possibility remained that the differences observed between transformation events expressing the constructs was due to environmental differences occurring between different experiments. To eliminate this possibility, three of the highest expressing events from the three sets of *Arabidopsis* transformation events were grown together and reanalyzed. This analyses confirmed the earlier differences between the sets of transformation events.

### SUMMARY OF EFFECTS OF THE ARTIFICIAL MAR ON TRANSGENE EXPRESSION

In both rice and *Arabidopsis*, the average expression level of transformation events expressing the MAR-containing constructs expressed at a higher level than those lacking the MAR elements (Table 3). In *Arabidopsis*, orientation of the MARs tested influenced the level of expression. Plants containing constructs in which the MARs were in the same orientation on either side of the GUS gene construct expressed GUS at higher levels than plants containing constructs in which the MARs were oriented in opposite orientations.

There appeared to be a proportionally higher expression level in the higher expressors than in the lower expressors in each set of transformation events. To document this, expression of the upper quartile of the expressors was compared (Table 3). Based on this data, the upper quartile of the transformation events expressing the AfAct2Af construct produce GUS protein at levels 5.6 times higher than in the upper quartile of events expressing the Actbin construct. The artificial MARs in opposite orientations in both rice and *Arabidopsis* enhance expression approximately two-fold over the respective constructs lacking MARs.

**Table 3**

| Comparison of the expression of transformation events expressing either the base constructs or base constructs flanked by the artificial MARs. | | | | | |
|---|---|---|---|---|---|
| | CONSTRUCT | | | | |
| | GOS2 | ArGOS2Af | ActBin | Ar-Act-Af | Af-Act-Af |
| species transformed | rice | rice | *Arabidopsis* | *Arabidopsis* | *Arabidopsis* |
| method of transformation | particle bombardment | particle bombardment | *Agrobacterium* | *Agrobacterium* | *Agrobacterium* |
| number of transformation events analyzed | 28 | 25 | 28 | 21 | 22 |
| average | 357 | 713 | 2,313 | 3,673 | 9,042 |
| median | 95 | 317 | 2,205 | 2,772 | 5,730 |
| upper quartile range | 258-3,076 | 1,674-2,661 | 3,047-5,311 | 6,240-10,614 | 15,595-27,179 |
| upper quartile average | 1,169 | 2,099 | 4,056 | 7,812 | 22,688 |
| relative average expression | 1.0 | 2.0 | 1.0 | 1.6 | 4.0 |
| relative upper average quartile expression | 1.0 | 1.8 | 1.0 | 1.9 | 5.6 |
| averages for the upper quartile of transformation event expression levels were statistically different (p is less than or equal to 0.05) based on t-test analyses. (Gopal K. Kanji 1995) | | | | | |

Previous published studies (reviewed by Holmes-Davis and Comai, 1998) have shown that, with the exception of one MAR, all MARs tested to date enhance expression of reporter genes. This study represents the first report that a MAR constructed using elements found preferentially in MARs can enhance expression in plant species representing both monocotyledonous and dicotyledonous plants.

### REFERENCES

**Allen, G.C., Hall, G.,Jr., Michalowski, S., Newman, W., Spiker, S., Weissinger, A.K. and Thompson, W.F.** (1996) High-level transgene expression in plant cells: Effects of a strong scaffold attachment region from tobacco. *Plant Cell* **8,** 899-913.
**Allen, G.C., Hall, G.E.,Jr., Childs, L.C., Weissinger, A.K., Spiker, S. and Thompson, W.F.** (1993) Scaffold attachment regions increase reporter gene expression in stably transformed plant cells. *Plant Cell* **5,** 603-613.
**An, Y.-Q., McDowell, J.M., Huang, S., McKinney, E.C., Chambliss, S. and Meagher, R.B.** (1996) Strong constitutive expression of the *Arabidopsis ACT2*/*ACT8* actin subclass in vegetative tissues. *Plant J*. **10,** 107-121.
**Avramova, Z. and Bennetzen, J.L.** (1993) Isolation of matrices from maize leaf nuclei: identification of a matrix-binding site adjacent to the Adh 1 gene. *Plant Mol. Biol*. **22,** 1135-1143.
**Avramova, Z., SanMiguel, P., Georgieva, E. and Bennetzen, J.L.** (1995) Matrix attachment regions and transcribed sequences within a long chromosomal continuum containing maize Adhl. *Plant Cell* **7,** 1667-1680.
**Bode, J., Kohwi, Y., Dickinson, L., Joh, Y., Klehr, D., Mielke, C. and Kohwi-Shigematsu, T.** (1992) Biological significance of unwinding capability of nuclear matrix-associated DNAs. *Science* **255,** 195-197.
**Bode, J., Schlake, T., Rios-Ramirez, M., Mielke, C., Stengert, M., Kay, V. and Klehr-Wirth, D.** (1995) Scaffold/matrix-attached regions: Structural properties creating transcriptionally active loci. *Int. Rev. of Cytol*. **162A,** 389-454.
**Bonifer, C., Vidal, M., Grosveld, F. and Sippel, A.E.** (1990) Tissue specific and position independent expression of the complete gene domain for chicken lysozyme in transgenic mice. *EMBO J*. **9,** 2843-2848.
**Boulikas, T.** (1995) Chromatin domains and the prediction of MAR sequences. *Int. Rev. of Cytol*. **162A,** 279-388.
**Boulikas, T. and Kong, C.F.** (1993) Multitude of inverted repeats characterize a class of anchorage sites of chromatin loops to the nuclear matrix. *J. Cell. Biochem*. **53,** 1-12.
**Bradford, M.M.** (1976) A rapid and sensitive method for the quantitation of microgram quantities of protein utilizing the principle of protein-dye binding. *Anal. Biochem*. **72,** 248-254.
**Breyne, P., Van Montagu, M., Depicker, A. and Gheysen, G.** (1992) Characterization of a plant scaffold attachment region in a DNA fragment that normalizes transgene expression in tobacco. *Plant Cell* **4,** 463-471.
**Breyne, P., Van Montagu, M. and Gheysen, G.** (1994) The role of scaffold attachment regions in the structural and functional organization of plant chromatin. *Transgenic Res*. **3,** 195-202.
**Buhrmester, H., von Kries, J.P. and Stratling, W.H.** (1995) Nuclear matrix protein ARBP recognizes a novel DNA sequence motif with high affinity. *Biochemistry* **34,** 4108-4117.
De Pater, B.S., van der Mark, F., Rueb, S., Katagiri, F., **Chua, N-H., Schilperoort, R.A. and Hensgens, L.A.M.** (1992) The promoter of the rice gene GOS2 is active in various different monocot tissues and binds rice nuclear factor ASF-1. *Plant J*. **2,** 837-844.
**Dennis, E.S., Gerlach, W.L., Pryor, A.J., Bennetzen, J.L., Inglis, A., Llewellyn, D., Sachs, M.M., Ferl, R.J. and Peacock, W.J.** (1984) Molecular analysis of the alcohol dehydrogenase (*Adh1*) gene of maize. *Nucl. Acids Res*. **12,** 3983-4000.
**Depicker, A., Stachel, S., Dhaese, P., Zambryski, P. and Goodman, H.M.** (1982) Nopaline synthase: Transcript mapping and DNA sequence. *J. Mol. Appl. Genet*. **1,** 561-573.
**Dickinson, L.A., Joh, T., Kohwi, Y. and Kohwi-Shigematsu, T.** (1992) A tissue-specific MAR/SAR DNA-binding protein with unusual binding site recognition. *Cell* **70,** 631-645.
**Dillon, N. and Grosveld, F.** (1994) Chromatin domains as potential units of eukaryotic gene function. *Curr Opinion Gen Dev* **4,** 260-264.
**Eggert, H. and Jack, R.S.** (1991) An ectopic copy of the *Drosophila ftz* associated SAR neither reorganizes local chromatin structure nor hinders elution of a chromatin fragment from isolated nuclei. *EMBO J*. **10,** 1237-1243.
**Forrester, W.C., van Genderen, C., Jenuwein, T. and Grosschedl, R.** (1994) Dependence of enhancer-mediated transcription of the immunoglobulin m gene on-nuclear matrix attachment regions. *Science* **265,** 1221-1225.
**Franck, A., Guilley, H., Jonard, G., Richards, K. and Hirth, L.** (1980) Nucleotide sequence of cauliflower mosaic virus DNA. *Cell* **21,** 285-294.
**Gritz, L. and Davies, J.** (1983) Plasmid-encoded hygromycin B resistance: the sequence of hygromycin B phosphotransferase gene and its expression in *Escherichia coli* and *Saccharomyces cerevisiae. Gene* **25,** 179-188.
**Hall, G.,Jr., Allen, G.C., Loer, D.S., Thompson, W.F. and Spiker, S.** (1991) Nuclear scaffolds and scaffold-attachment regions in higher plants. Proc. *Natl Acad. Sci. USA* **88,** 9320-9324.
**Jackson, D.A.** (1995) Nuclear organization: uniting replication foci, chromatin domains and chromosome structure. *Bioessays* **17,** 587-591.
**Jefferson, R.A.** (1987) Assaying chimeric genes in plants: The GUS gene fusion system. *Plant Mol. Biol*. *Rep*. **5,** 387-405.
**Jefferson, R.A., Burgess, S.M. and Hirsh, D.** (1986) b-Glucuronidase from *Escherichia coli* as a gene-fusion marker. *Proc. Natl Acad. Sci. USA* **83,** 8447-8451.
**Jefferson, R.A., Kavanagh, T.A. and Bevan, M.W.** (1987) GUS fusions: b-glucuronidase as a sensitive and versatile gene fusion marker in higher plants. *EMBO J*. **6,** 3901-3907.
Kanji, G. K., (1995) *100 Statistical Tests*, Sage Publications Inc., Thousand Oaks CA
**Kriz, A.L., Boston, R.S. and Larkins, B.A.** (1987) Structural and transcriptional analysis of DNA sequences flanking genes that encode 19 kilodalton zeins. *Mol. Gen. Genet*. **207(1),** 90-98.
**Lewin, B.** (1994) Chromatin and gene expression: Constant questions, but changing answers. *Cell* **79,** 397-406.
**McKnight, R.A., Shamay, A., Sankaran, L., Wall, R.J. and Hennighausen, L.** (1992) Matrix-attachment regions can impart position-independent regulation of a tissue-specific gene in transgenic mice. *Proc. Natl Acad. Sci*. *USA* **89,** 6943-6947.
**Mlynarova, L., Jansen, R.C., Conner, A.J., Stiekema, W.J. and Nap, J-P.** (1995) The MAR-mediated reduction in position effect can be uncoupled from copy number-dependent expression in transgenic plants. *Plant Cell* **7,** 599-609.
**Mlynarova, L., Loonen, A., Heldens, J., Jansen, R.C., Keizer, P., Stiekema, W.J. and Nap, J-P.** (1994) Reduced position effect in mature transgenic plants conferred by the chicken lysozyme matrix-attachment region. *Plant Cell* **6,** 417-426.
**Morrison, D.A., Trombe, M.C., Hayden, M.K., Waszak, G.A. and Chen, J.** (1984) Isolation of transformation-deficient Streptococcus pneumoniae mutants defective in control of competence, using insertion-duplication mutagenesis with the erythromycin reistance determinant of pAMb1. *J. Bacteriol*. **159,** 870-876.
**Mullineaux, P.M., Donson, J., Morris-Krsinich, B.A.M., Boulton, M.I. and Davies, J.W.** (1984) The nucleotide sequence of maize streak virus DNA. *EMBO J*. **3,** 3063-3068.
**Nagagomi, K., Kohwi, Y., Dickinson, L.A. and Kohwi-Shigematsu, T.** (1994) A novel DNA binding motif in the nuclear matrix attachment DNA-binding protein SATB1. *Mol. Cell. Biol*. **14,** 1852-1860.
**Neznanov, N., Kohwi-Shigematsu, T. and Oshima, R.G.** (1996) Contrasting effects of the SATB1 core nuclear matrix attachment region and flanking sequences of the keratin 18 gene in transgenic mice. *Mol. Biol. Cell* **7,** 541-552.
**Quigley, F., Brinkmann, H., Martin, W.F. and Cerff, R.** (1989) Strong functional GC pressure in a light-regulated maize gene encoding subunit GAPA of chloroplast glyceraldehyde-3-phosphate dehydrogenase: implications for the evolution of GAPA pseudogenes. *J*. *Molec. Evol*. **29(5),** 412-421.
**Sander, M. and Hsieh, T-S.** (1985) Drosophila topoisomerase II double-strand cleavage: Analysis of DNA sequence homology at the cleavage site. *Nucl*. *Acids Res*. **13,** 1057-1072.
**Schöffl, F., Schroder, G., Kliem, M. and Rieping, M.** (1993) An SAR sequence containing 395 bp DNA fragment mediates enhanced, gene-dosage-correlated expression of a chimaeric heat shock gene in transgenic tobacco plants. *Transgenic Res*. **2,** 93-100.
**Siebert, P.D., Chenchik, A., Kellogg, D.E., Lukyanov, R.A. and Lukyanov, A.S.** (1995) An improved PCR method for walking in uncloned genomic DNA. *Nucl. Acids Res*. **23,** 1087-1088.
**Slatter, R.E., Dupree, P. and Gray, J.C.** (1991) A. scaffold-associated DNA region is located downstream of the pea plastocyanin gene. *Plant Cell* **3,** 1239-1250.
**Southern, E.** (1975) Detection of specific sequences among DNA fragments separated by gel electrophoresis. *J*. *Mol. Biol*. **98,** 503.
**Southern, E.** (1979) Gel electrophoresis of restriction fragments. *Methods of Enzymol*. **68,** 152.
**Spiker, S. and Thompson, W.F.** (1996) Nuclear matrix attachment regions and transgene expression in plants. *Plant Physiol*. **110,** 15-21.
**Stein, G.S., Lian, J.B., Dworetzky, S.I., Owen, T.A., Bortell,** R., Bidwell, J.P. and van Wijnen, A.J. (1991) Regulation of transcription-factor activity during growth and differentiation: Involvement of the nuclear matrix in concentration and localization of promoter binding proteins. *J. Cell. Biochem*. **47,** 300-305.
**Stief, A., Winter, D.M., Stratling, W.H. and Sippel, A.E.** (1989) A nuclear DNA attachment element mediates elevated and position-independent gene activity. *Nature* **341,** 343-345.
**Travers, A.A.** (1990) Why bend DNA. *Cell* **60,** 177-180.
**Van der Geest, A.H.M., Hall, G.E.,Jr., Spiker, S. and Hall,** T.C. (1994) The b-phaseolin gene is flanked by matrix attachment regions. *Plant J*. **6,** 413-423.
**Van der Geest, A.H.M. and Petolino, J.F.** (1998) Expression of a modified green fluorescent protein gene in transgenic maize plants and progeny. *Plant Cell Rep*. **17,** 760-764.
**von Kries, J.P., Buhrmester, H. and Stratling, W.H.** (1991) A matrix/scaffold attachment region binding protein; Identification, purification and mede of binding. *Cell* **64,** 123-135.
**Wolffe, A.P.** (1994) The transcription of chromatin templates. *Curr Opinion Gen Dev* **4,** 245-254.
**Yanisch-Perron, C., Vieira, J, and Messing, J.** (1985) Improved M13 phage cloning vectors and host strains: Nucleotide sequences of the M13mp18 and pUC 19 vectors. Gene 33, 103-119.

## Claims

1. An isolated DNA molecule comprising bp 11 to 309 of SEQ ID NO: 1.

2. A DNA construct comprising, in the 5' to 3' direction: a transcription initiation region functional in plant cells, a structural gene operatively associated with the transcription initiation region, a 3' untranslated region, and a matrix attachment region comprised of bp 11 to 309 of SEQ ID NO: 1 positioned either 5' to said transcription initiation region or 3' to said structural gene.

3. A DNA construct of claim 2 wherein said matrix attachment region comprises two or more tandem copies of bp 11 to 309 of SEQ ID NO:1.

4. A DNA construct of claim 2 wherein a first matrix attachment region comprised of bp 11 to 309 of SEQ ID NO: 1 is positioned 5' to said transcription initiation region and a second matrix attachment region comprised of bp 11 to 309 of SEQ ID NO: 1 is positioned 3' to said structural gene.

5. A DNA construct of claim 4 wherein each of said matrix attachment regions is comprised of two or more tandem copies of bp 11 to 309 of SEQ ID NO:1.

6. A method of making recombinant plant cells having increased expression of structural genes introduced therein which comprises:
transforming a plant cell capable of regeneration with a DNA construct of claim 2.

7. A method of claim 6 wherein the matrix attachment region in said DNA construct comprises two or more tandem copies of bp 11 to 309 of SEQ ID NO:1.

8. A method of claim 6 wherein the DNA construct includes a first matrix attachment region comprised of bp 11 to 309 of SEQ ID NO: 1 positioned 5' to said transcription initiation region and a second matrix attachment region comprised of bp 11 to 309 of SEQ ID NO: 1 positioned 3' to said structural gene.

9. A method of claim 8 wherein said first and second matrix attachment regions are each comprised of two or more tandem copies of bp 11 to 309 of SEQ ID NO:1.

10. A transformed plant cell containing DNA of bp 11 to 309 of SEQ ID NO:1.

## Patentansprüche

1. Isoliertes DNA-Molekül, umfassend bp 11 bis 309 von SEQ ID NO:1.

2. DNA-Konstrukt, umfassend in der Richtung 5' nach 3': eine in Pflanzenzellen funktionelle Transkriptionsinitiationsregion, ein Strukturgen, welches operativ mit der Transkriptionsinitiationsregion verbunden ist, eine 3' nicht translatierte Region und eine Matrix-Anheftungsregion, umfassend bp 11 bis 309 von SEQ ID NO: 1 entweder in 5'-Position bezüglich der Transkriptionsinitiationsregion oder in 3'-Position bezüglich des Strukturgens.

3. DNA-Konstrukt nach Anspruch 2, worin die Matrix-Anheftungsregion zwei oder mehrere Tandemkopien von bp 11 bis 309 von SEQ ID NO: 1 umfasst.

4. DNA-Konstrukt nach Anspruch 2, worin eine erste Matrix-Anheftungsregion, welche bp 11 bis 309 von SEQ ID NO:1 umfasst, in 5'-Position bezüglich der Transkriptionsinitiationsregion angeordnet ist, und eine zweite Matrix-Anheftungsregion, welche bp 11 bis 309 von SEQ ID NO:1 umfasst, in 3'-Position bezüglich des Strukturgens angeordnet ist.

5. DNA-Konstrukt nach Anspruch 4, worin jede der Matrix-Anheftungsregionen zwei oder mehrere Tandemkopien von bp 11 bis 309 von SEQ ID NO: 1 umfasst.

6. Verfahren zur Herstellung rekombinanter Pflanzenzellen mit einer erhöhten Expression der darin eingebrachten Strukturgene, welches umfasst: Transformieren einer zur Regeneration befähigten Pflanzenzelle mit einem DNA-Konstrukt nach Anspruch 2.

7. Verfahren nach Anspruch 6, worin die Matrix-Anheftungsregion in dem DNA-Konstrukt zwei oder mehrere Tandemkopien von bp 11 bis 309 von SEQ ID NO: 1 umfasst.

8. Verfahren nach Anspruch 6, worin das DNA-Konstrukt eine erste Matrix-Anheftungsregion, welche bp 11 bis 309 von SEQ ID NO:1 umfasst, in 5'-Position bezüglich der Transkriptionsinitiationsregion umfasst, und eine zweite Matrix-Anheftungsregion, welche bp 11 bis 309 von SEQ ID NO:1 umfasst, in 3'-Position bezüglich des Strukturgens umfasst.

9. Verfahren nach Anspruch 8, worin die erste und zweite Matrix-Anheftungsregion jeweils zwei oder mehrere Tandemkopien von bp 11 bis 309 von SEQ ID NO: 1 umfassen.

10. Transformierte Pflanzenzelle, welche die DNA von bp 11 bis 309 von SEQ ID NO:1 umfasst.

## Revendications

1. Molécule d'ADN isolé, comprenant les paires de bases n° 11 à 309 de la séquence n° 1.

2. Construction d'ADN comprenant, dans le sens 5' → 3' : une région de démarrage de transcription, fonctionnelle chez les cellules végétales ; un gène de structure, opérationnellement associé à la région de démarrage de transcription ; une région 3' non traduite ; et une région de liaison à la matrice, comprenant les paires de bases n° 11 à 309 de la séquence n° 1 et placée soit en 5' de ladite région de démarrage de transcription, soit en 3' dudit gène de structure.

3. Construction d'ADN conforme à la revendication 2, dans laquelle ladite région de liaison à la matrice comprend deux copies en tandem ou plus des paires de bases n° 11 à 309 de la séquence n° 1.

4. Construction d'ADN conforme à la revendication 2, dans laquelle une première région de liaison à la matrice, comprenant les paires de bases n° 11 à 309 de la séquence n° 1, est placée en 5' de ladite région de démarrage de transcription, et une deuxième région de liaison à la matrice, comprenant les paires de bases n° 11 à 309 de la séquence n° 1, est placée en 3' dudit gène de structure.

5. Construction d'ADN conforme à la revendication 4, dans laquelle chacune desdites régions de liaison à la matrice comprend deux copies en tandem ou plus des paires de bases n° 11 à 309 de la séquence n° 1.

6. Procédé de préparation de cellules végétales recombinantes chez lesquelles l'expression de gènes de structure introduits dans ces cellules est accrue, lequel procédé comporte le fait de transformer, avec une construction d'ADN conforme à la revendication 2, une cellule végétale capable de se régénérer.

7. Procédé conforme à la revendication 6, dans lequel, dans ladite construction d'ADN, la région de liaison à la matrice comprend deux copies en tandem ou plus des paires de bases n° 11 à 309 de la séquence n° 1.

8. Procédé conforme à la revendication 6, dans lequel la construction d'ADN comporte une première région de liaison à la matrice, comprenant les paires de bases n° 11 à 309 de la séquence n° 1 et placée en 5' de ladite région de démarrage de transcription, et une deuxième région de liaison à la matrice, comprenant les paires de bases n° 11 à 309 de la séquence n° 1 et placée en 3' dudit gène de structure.

9. Procédé conforme à la revendication 8, dans lequel lesdites première et deuxième régions de liaison à la matrice comprennent chacune deux copies en tandem ou plus des paires de bases n° 11 à 309 de la séquence n° 1.

10. Cellule végétale transformée, contenant un ADN qui comprend les paires de bases n° 11 à 309 de la séquence n° 1.
